Europäisches Patentamt

European Patent Office

Office européen des brevets

(11) Publication number: **0 313 224**
**A1**

(12) **EUROPEAN PATENT APPLICATION**

(21) Application number: 88308926.0

(22) Date of filing: 27.09.88

(51) Int. Cl.4: **C12N 5/00 , C07K 15/00 ,**
**G01N 33/574**

(30) Priority: 28.09.87 US 101572

(43) Date of publication of application:
26.04.89 Bulletin 89/17

(84) Designated Contracting States:
**AT BE CH DE ES FR GB GR IT LI LU NL SE**

(71) Applicant: **CHILDREN'S HOSPITAL MEDICAL**
**CENTER**
**Elland and Bethesda Avenues**
**Cincinnati Ohio 45229(US)**

Applicant: **THE UNITED STATES OF AMERICA**
**as represented by the Secretary U.S.**
**Department of Commerce**
**National Technical Information Service**
**Office of Government Inventions and**
**Patents 5285 Port Royal Road**
**Springfield, Virginia 22161(US)**

(72) Inventor: **Whitsett, Jeffrey A.**
**5565 Salem Road**
**Cincinnati Ohio 45230(US)**
Inventor: **Gazdar, Adi F.**
**10414 Windsor View Drive**
**Potomac Maryland 20854(US)**

(74) Representative: **Allam, Peter Clerk et al**
**LLOYD WISE, TREGEAR & CO. Norman**
**House 105-109 Strand**
**London WC2R 0AE(GB)**

(54) **Human cell lines of epithelial lung adenocarcinoma origin, human proteins and methods.**

(57) The present invention relates to novel continuous human adenocarcinoma cell lines of epithelial lung tissue origin that produce fully processed or semi-processed surfactant-associated proteins, e.g., SAP-35 and SPL(Phe). The surfactant-associated proteins confer biological activity to pulmonary surfactant phospholipids and are useful for formulating pulmonary surfactant replacement for, inter alia, diagnosis and therapy of human diseases associated with surfactant deficiency. Exemplary cell lines of the present invention designated as NCI-H441-4, NCI-H820, NCI-H322 and NCI-H1404 are deposited with the American Type Culture Collection (ATCC) of Rockville, Maryland under accession Nos. CRL 9400, CRL 9531, CRL 9532 and CRL 9533, respectively.

The present invention also relates to a novel method for regulating the expression of a surfactant-associated protein by cells having the ability to express at least one surfactant-associated protein and/or mRNA encoding therefore by exposing the cells to a regulating agent, such as a glucocorticoid hormone, 8-Br-cAMP or a functional equivalent, to selectively induce or inhibit the expression of a surfactant-associated protein. The present invention is also directed to novel methods to screen for tumor cells of pulmonary epithelial origin which produce surfactant-associated proteins and purifying the proteins from lysates of the cells or the media. The present invention also relates to novel human proteins, $M_r = 40-46,000$, $M_r = 27-33,000$ and $M_r = 23,000$ on SDS-PAGE, which are believed to be precursors of the SPL(Phe) human protein. The SPL(Phe) mRNA initially translates an $Mr = 40\text{-}42,000$ preproprotein which is glycosylated at asparagine residues and sialylated resulting in an $Mr = 40\text{-}46,000$ SPL(Phe) proprotein. The $Mr = 27\text{-}33,000$ protein is a proteolytically cleaved fragment of the $Mr = 40\text{-}46,000$ precursor, and the $Mr = 23,000$ protein results from de-glycosylating and de-sialylating the $Mr = 27\text{-}33,000$ protein.

# HUMAN CELL LINES OF EPITHELIAL LUNG ADENOCARCINOMA ORIGIN, HUMAN PROTEINS AND METH-ODS

## Field of the Invention

The present invention relates to novel established and continuous human adenocarcinoma cell lines of epithelial lung tissue origin that produce pulmonary surfactant-associated proteins. The present invention also relates to novel methods for regulating the expression of pulmonary surfactant-associated proteins, novel methods which screen for tumor cells of pulmonary epithelial origin that produce pulmonary surfactant-associated proteins, and novel methods for harvesting produced surfactant-associated proteins. The present invention further relates to novel human pulmonary surfactant-associated proteins.

## Background

Pulmonary surfactant is a lipoprotein complex synthesized and secreted into alveoli of the lung by Type II epithelial lung cells. Although greater than 85% of pulmonary surfactant is lipid, several surfactant-associated proteins have been identified as being associated with the biophysical properties necessary for surfactant function. The most abundant surfactant-associated protein is a glycoprotein of $M_r = 35,000$, referred to as SAP-35, which has been isolated from lung lavage in many species. The genes and cDNA for human and canine SAP-35 encode an approximately 23,000 Dalton polypeptide, containing a collagen-like Gly-X-Y-Gly domain. The size and charge heterogeneity observed in both in vivo and in vitro Type II cell cultures is due in part to the addition of asparagine-linked carbohydrate, sulfation, carboxylation, acetylation and hydroxylation; other post-translational processing is also possible including addition of covalent fatty acids, such as palmitate or myristate, to the SAP-35 polypeptide. SAP-35 induces calcium-dependent phospholipid aggregation and enhances some biophysical properties of surfactant phospholipids.

Recently, two smaller surfactant-associated proteins, SPL(Phe) and SPL(pVal), have been identified in organic solvent extracts of pulmonary surfactant lipids. These are hydrophobic 5-12,000 Dalton polypeptides, tightly associated with surfactant phospholipids. Both polypeptides are components of active pulmonary surfactant and have been associated with enhanced biophysical activity of surfactant phospholipids. Identification of cDNAs and in vitro translation of lung mRNA has demonstrated that these polypeptides are synthesized as larger precursors.

Pulmonary surfactant is critically necessary for efficient breathing. Certain disease states, such as hyaline membrane disease of neonates, are characterized by surfactant deficiency. Since both SAP-35 and the surfactant proteolipids SPL(Phe) and SPL(pVal) improve the biophysical surfactant properties of phospholipids, these proteins are expected to be useful in the therapy of surfactant deficiency.

Notwithstanding the importance of these proteins to pulmonary surfactant, acquiring therapeutically useful quantities presents a formidable problem. One potential solution to the problem is to extract the pulmonary surfactant-associated proteins from human cadaver lungs. Such material, however, is in short supply. Moreover, cadaver lungs are subject to contamination by human viruses and bacterial endotoxins, which make the isolated proteins unsuitable for human therapy. Even if the cadaver lungs were not contaminated, this approach is unacceptable since the yields generated are extremely small and purification is difficult due to the fact that the proteins are present in such material in very low quantities.

Another possible solution to the difficulties associated with obtaining human surfactant-associated proteins is to isolate human Type II epithelial cells, grow them in cell culture, and harvest the surfactant-associated proteins from the cells or medium. Although this approach also appears to be initially promising, particularly in view of the facts that Type II lung cells produce several fully processed surfactant-associated proteins and human lung epithelial cells can be easily obtained from minced fresh human lung material, lung biopsy material or aborted fetuses, it too is not without its shortcomings. For instance, explant cultures containing Type II epithelial cells in culture generally do not secrete surfactant-associated proteins into medium. Consequently, such cells must be harvested and lysed in order to recover the proteins. This can severly limit the yields attainable. In addition, Type II epithelial cells do not continue to reproduce in culture, and they lose differentiated features within about 24-48 hours in culture. As a consequence of de-differentiation, they cease producing surfactant proteins. These difficulties may be substantially overcome if human Type II epithelial cells could be established in culture; that is, if they could be modified to enable reproduction (hence increase in number) and prevent de-differentiation so that they retain their ability to synthesize surfactant-associated proteins over long periods of time. However, no methodology to date has been demonstrated which permits Type II epithelial cells (human or animal) to be established in culture with long-term production of the surfactant-associated proteins. Nor has there been establishment of Type II epithelial human or animal cells in culture medium over long periods of time in which such cells have retained their differentiation and ability to produce surfactant-associated proteins.

Still another potential solution to overcoming the problem associated with obtaining human surfactant-associated proteins is to use recombinant DNA technology. With this solution, genes coding for selected surfactant-associated proteins would be isolated and inserted into a suitable cloning vector, such as a plasmid or phage. This vector could then be used to transfect host cells, such as bacteria, yeast or mammalian cells, like Chinese hamster ovary CHO cells, which could then be cultured for expression of the protein of interest. There are problems, however, that also limit the usefulness of this approach. First, the level of expression of the introduced gene is often low. Second, the surfactant-associated proteins must be extensively modified post-translationally before they attain full biological activity. In the case of SAP-35, processing involves hydroxylation of proline residues, addition of asparagine-linked carbohydrate, acylation with fatty acids, and carbohydrate sulfation. All of these post translational modifications are accomplished spontaneously by Type II epithelial cells. Genetically engineered non-Type II epithelial cells, such as bacteria or yeast cells, however, are unlikely to perform all of the necessary post-translational modifications required to produce mature surfactant-associated proteins which are indistinguishable from the human proteins. The biochemical machinery for such modifications is complex and may be specific to the Type II human cells. Thus, expression of human recombinant DNA pulmonary surfactant-associated genes in non-mammalian cells will not in general give rise to proteins which are truly identical to the human surfactant-associated proteins. Third, even if it were feasible for cells to be genetically constructed to express human surfactant-associated proteins in high abundance with identity to the natural molecules, these cells would ordinarily be engineered to express only one surfactant-associated protein. In contrast, the Type II cells synthesize and secrete several surfactant-associated proteins in high abundance.

The difficulties associated with obtaining human surfactant-associated proteins have caused the manufacturers of a recently introduced therapeutic surfactant preparation (such as surfactant-TA, Abbott Pharmaceutical) to use surfactant-associated proteins isolated from alternative sources, such as cow lungs, in preference to proteins isolated from human sources. The cow surfactant-associated proteins, however, are not identical to human proteins. Human proteins clearly are preferable, but the practical difficulties in isolating human surfactant-associated proteins from human cadavers, cultured Type II epithelial cells and genetically engineered cells as outlined above may render these approaches at least from a commercial standpoint unacceptable.

Thus, the establishment of a naturally occurring human cell line which can express and secrete surfactant-associated proteins would be extremely advantageous. It would also be very advantageous to have means available for inducing an established human cell line to express surfactant-associated proteins in generous quantities to overcome the above mentioned problems associated with obtaining human surfactant-associated proteins.

Summary of the Invention

In brief, the present invention alleviates the above mentioned problems and shortcomings of the present state of the art through the discovery of novel continuous human tumor cell lines that are morphologically similar to Type II epithelial lung cells and capable of producing and secreting at least one surfactant-associated protein. Moreover, at least one of the cell lines of the present invention is capable of producing at least one surfactant-associated protein in generous quantities following inducement.

The established cell lines of the present invention are generally obtained from epithelial lung adenocarcinoma tissue removed surgically from human patients diagnosed as having lung carcinoma. Histologically, the cell lines are typical of "non-small" cell lung carcinoma. The cell lines form epithelial-like cells in culture which are generally loosely adherent on plastic and grow in monolayers in culture or in floating aggregates. The cells of the NCI-H441-4 and NCI-H820 cell lines have morphologic criteria typical of Type II epithelial cells and contain lamellar or lipoidal-like body inclusions confirmed by phase contrast microscopy and electron microscopic examination (not shown). In addition, the cells have numerous granules and lipoidal, refractile bodies typical of surfactant lipid inclusions. These characteristics are similar to those observed in Type II epithelial cells in primary cultures from rat and human. The cells of the NCI-H322 cell line are epithelial cells with numerous granular inclusions. The cells of the cell lines are believed to be homogeneous and have been successfully passaged in cell culture for more than one year. The cell lines presently exist as stable, continuous cell lines.

An established cell line in accordance with this invention is designated as NCI-H441-4 and is deposited with the American Type Culture Collection (ATCC) of Rockville, Maryland, under accession number ATCC CRL 9400. Other established cell lines in accordance with this invention are designated as NCI-H820, NCI-H322 and NCI-H1404 and are also deposited with the ATCC of Rockville, Maryland under accession numbers CRL 9531, CRL 9532 and CRL 9533, respectively. Although this indicated public availability is the simplest method of obtaining the NCI-H441-4 cell line or the other three exemplary cell lines in accordance with this invention, it is not altogether impossible or improbable that similar and functionally substantially identical human cell lines might be produced continuously by other methods in view of the teachings of this invention. Such functionally substantially identical cell lines are considered to be biologically equivalent to the cell lines of this invention and therefore are within the general scope of the present invention. Also, such substantially identical cell lines, which can be obtained by those skilled in the art by modifying, cloning or subcloning the cell lines described and thereby provided by the present invention, without substantially altering the morphological and functional properties of the cell lines or those cultivated, are within the scope of the present invention.

The cells of, for example, the NCI-H441-4, NCI-H820, NCI-H1404 and NCI-H322 cell lines appear to produce and/or secrete at least one surfactant-associated protein, such as an SAP-35 and SPL-(Phe) protein. The surfactant-associated proteins produced by the cell lines may be in a semi-processed or fully processed form and are believed to be indistinguishable from those surfactant-associated proteins produced and secreted by normal human pulmonary Type II epithelial cells. The cell lines of the present invention therefore overcome the problems discussed above by providing a continuous source of human surfactant-associated proteins which can be isolated by the methods described herein or by conventional methods. In addition, due to the constitutive production of the surfactant-associated proteins, the cells can provide a source for the RNA or DNA encoding the proteins of interest as well as for some or all of the enzymes which are responsible for the post-translational processing of the proteins. The cells of the cell lines may also be cloned for purposes of obtaining high-producing clonal cell lines.

The present invention is further predicated in part upon the discovery of a novel method for selectively regulating production of a surfactant-associated protein and the mRNA encoding for the protein. This unique method contemplates exposing cultured cells having the ability to produce at least one such protein to one or more effective regulating agents to induce and/or inhibit the production of the selected protein. It is surprisingly found that when cultured cells, such as the NCI-H441-4 and NCI-820, are exposed to such an agent, the production of one or more proteins and/or the mRNAs encoding such proteins may be increased. It is also surprisingly found that the production of one or more of the other proteins and the mRNAs encoding the proteins may be simultaneously decreased. For example, when cells of the NCI-H441-4 cell line are cultivated in medium containing dexamethasone, the production of the precursors of the hydrophobic protein SPL(Phe) and the SPL(Phe) mRNA are remarkably increased whereas the production of the SAP-35 glycoprotein and the SAP-35 mRNA are significantly decreased. Likewise, when the cells of the NCI-H441-4 cell line are cultivated in a medium containing 8-Br-cAMP, production of SAP-35 increases significantly. It should be recognized, however, by those versed in this art that any cells cultured in medium and having the ability to produce the surfactant-associated proteins and the particular mRNAs encoding such proteins as well as having the ability to regulate production of the proteins may be regulated via the methods of this invention to selectively induce or inhibit production of at least one surfactant-associated protein and the particular mRNA encoding the protein. Moreover, it should be appreciated that a particular regulating agent may be capable of inducing or increasing production of a surfactant-associated protein while simultaneously inhibiting or decreasing production of another, as in the case with dexamethasone as discussed above.

The present invention is also predicated in part upon the discovery of novel human surfactant-associated proteins, $M_r$ = about 40-46,000, $M_r$ = about 27-33,000 and $M_r$ = about 23,000 on SDS-PAGE, which are believed to be precursors of the active or fully processed hydrophobic SPL(Phe) protein. The novel precursor proteins have been produced by the NCI-H441-4 cell line and isolated therefrom by, for instance, immunoprecipitation with a bovine polyclonal antibody reactive with SPL(Phe). The immunoprecipitated proteins are analyzed by 1D-SDS-PAGE and NEPHGE-SDS-PAGE (nonequilibrium pH gradient electrophoresis-sodium dodecylsulfate-polyacrylamide gel electrophoresis) analysis and autoradiography. The $M_r$ = 40-46,000 protein has a pI equal to about 4.6-5.4 and is believed to be expressed initially as a $M_r$ = 40-42,000 preproprotein that is glycosylated at the asparagine residues and sialylated to generate a proprotein of about 40-46,000 Daltons. The $M_r$ = 27-

33,000 protein has a pl of about 6.0-7.3 and is a proteolytically cleaved fragment of the Mr = 40-46,000 proprotein. The 27-33,000 Dalton protein is also glycosylated at the asparagine residues and sialylated. Both the Mr = 40-46,000 precursor and Mr = 27-33,000 proteolytically cleaved fragment are believed to be processed to the active Mr = 6-8,000 protein. Moreover, the Mr = 40-46,000 and Mr = 27-33,000 precursors are believed to be relatively more soluble in water than the smaller hydrophobic active Mr = 6-8,000 protein. The size and charge heterogeneity of the 40-46,000 Dalton proprotein and 27-33,000 Dalton protein are thought to be related in part to the presence of an asparagine-linked oligosaccharide. The Mr = 40-46,000 and Mr = 27-33,000 novel proteins are sensitive to endoglycosidase-F digestion and as stated above are reactive with antibody for human SPL(Phe). The 23,000 Dalton protein is a de-glycosylated and de-sialylated precursor of the active SPL(Phe) that is reactive with antibody for SPL(Phe) and is generated following enzymatic cleavage of asparagine-linked oligosaccharide on the 27-33,000 Dalton protein. The 23,000 Dalton protein has a pl of about 6.3-7.3.

The present invention is further predicated in part upon novel methods to screen for tumor cells of pulmonary epithelial origin that may constitutively produce a surfactant-associated protein, and a novel affinity purification method to harvest or purify the produced proteins from the cells and/or media. Quite surprisingly, the screening methods of the present invention screen positive for "non-small" or large cell type tumor cells of pulmonary epithelial origin with amazing accuracy while they screen negative for the small cell type lung tumors. Generally speaking, one screening method contemplated by the present invention involves exposing lysed tumor cells or media in which the tumor cells are cultured to an anti-surfactant-associated protein (antibody) to immunoprecipitate the protein therefrom and identifying the immunoprecipitated protein to confirm production by the tumor cells. Another screening method contemplated by the present invention involves exposing an mRNA encoding a surfactant-associated protein isolated from tumor cells of pulmonary epithelial origin to a radio-labelled cDNA probe complimentary to the mRNA, hybridizing the radio-labelled cDNA with the mRNA, and identifying the hybridized radio-labelled probe to confirm expression of the mRNA by the tumor cells. As to the novel affinity purification method, this method generally speaking involves substantially purifying surfactant-associated protein from cultured cells comprising applying medium in which the cells have been cultured or lysates of the cells to a column to which an affinity agent has been conjugated for binding with the protein and separating the bound protein from the column to generate the substantially purified protein. The affinity agent can be, for instance, an anti-surfactant-associated protein (polyclonal or monoclonal antibody) or -methyl-mannose. It should be understood that in practicing these methods any effective affinity agent may be utilized that is reactive with a surfactant-associated protein. Likewise, any effective cDNA probe that is complimentary to an mRNA encoding a surfactant-associated protein may be employed.

In the literature, there are preliminary reports of 'surfactant-associated glycoprotein' synthesis by A549 cells, a continuous cell line initiated from a bronchioalveolar cell carcinoma. See, Balis et al: Lab. Invest., 52(6), 657-669 (1985) and Balis et al: Exp. Lung. Res., 6:197-213 (1984). While it has been reported by Balis et al that rabbit antibodies raised against high molecular weight aggregates of surfactant-associated glycoprotein react not only with type II cells, but also with A549 cells, subsequent investigations have provided no substantial evidence corroborative of synthesis of SAP-35 by A549 cells. Most notably, no SAP-35 was detected using antibodies raised against SAP-35 upon screening of A549 cells and culture supernatant, even when 8-Br-cAMP was included in the cell growth medium. Moreover, no SAP-35 synthesis was detected after [$^{35}$S]-methionine labelling of A549 cells.

Also in the literature, it has been reported that SAP-35 protein and/or RNA synthesis in human fetal lung explant may be enhanced by 8-Br-cAMP or epidermal growth factor and inhibited by dexamethasone or transforming growth factor-beta. See Snyder, J.M. et al: Endocrinology, 120:1250-1257 (1987); Whitsett, J.A. et al: J. Biol. Chem., 262:5256-5261 (1987); Whitsett, J.A. et al: J. Biol. Chem., 262:7908-7913 (1987); Ballard, P.L. et al: Proc. Natl. Acad. Sci., U.S.A., 83:9527-9531 (1986); and Mendelson, C.R. et al: J. Biol. Chem., 261:9938-9943 (1986). However, it has been unknown heretofore that SAP-35 protein and/or RNA synthesis can be regulated, i.e., induced or inhibited, in human cultured cells by such regulating agents.

The surfactant-associated proteins produced by the cell lines of this invention or their fragments can be purified and used for the generation of monoclonal or polyclonal antibodies, as antigens for immunoassays for quantification of the proteins in biological fluids and the diagnosis of pulmonary maturity or other disease states of the lung, such as hyaline membrane disease. Antibodies and cDNAs derived from the proteins and mRNAs, respectively, for the surfactant-associated proteins can be used to screen and identify other tumor cells of pulmonary epithelial origin which produce the surfactant-associated proteins in accordance with the methods described herein.

As already indicated, the surfactant-associated proteins produced by the cell lines of this invention are believed to be indistinguishable from those surfactant-associated proteins that are obtained from normal human Type II epithelial cells. The proteins or their fragments produced by the cell lines of this invention can therefore be mixed alone or together in combinations with phospholipids to make synthetic surfactants for replacement therapy for treatment of hyaline membrane disease or other respiratory diseases. Such combinations, because of their surface properties and ability to rapidly spread, can further be used as carriers for delivery of other drugs or antibiotics to, for instance, the distal respiratory epithelium.

The above features and advantages of the present invention will be better understood with reference to the following accompanying figs., detailed description and example which are illustrative of preferred embodiments of the present invention.

Description of the Figs.

With reference to the accompanying figs. which are illustrative of the embodiments within the scope of this invention:

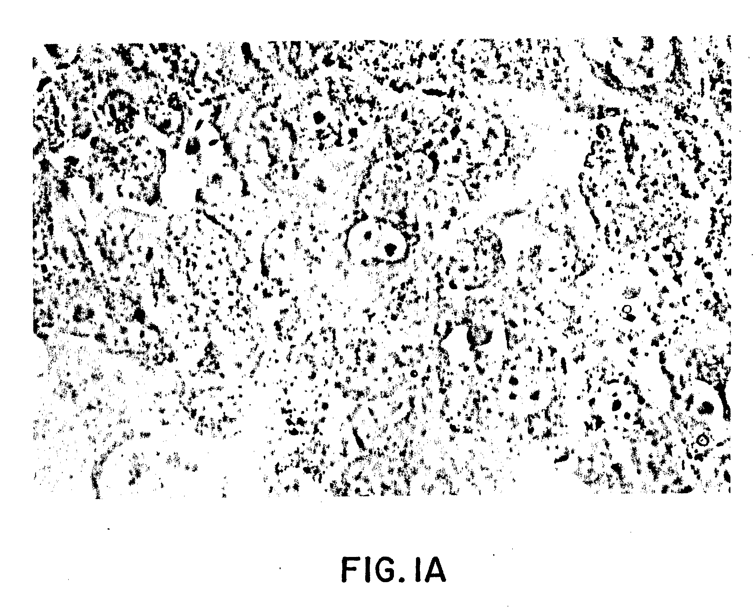

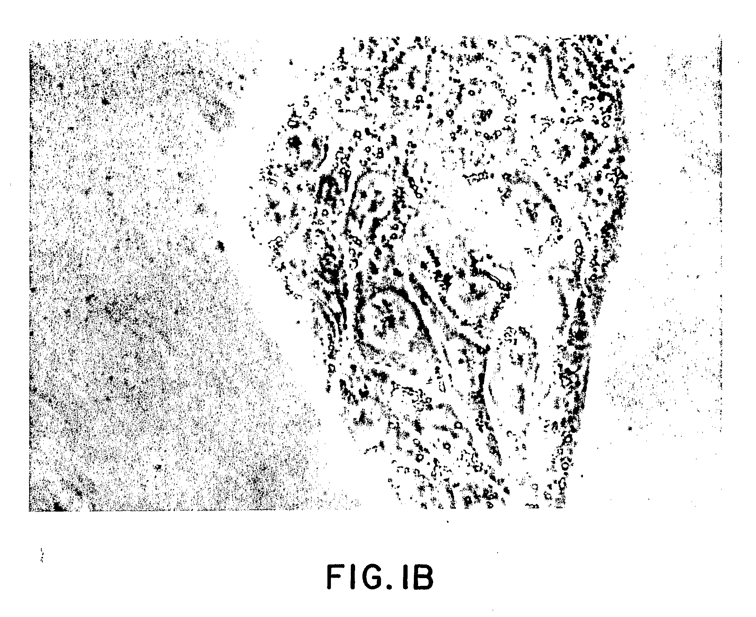

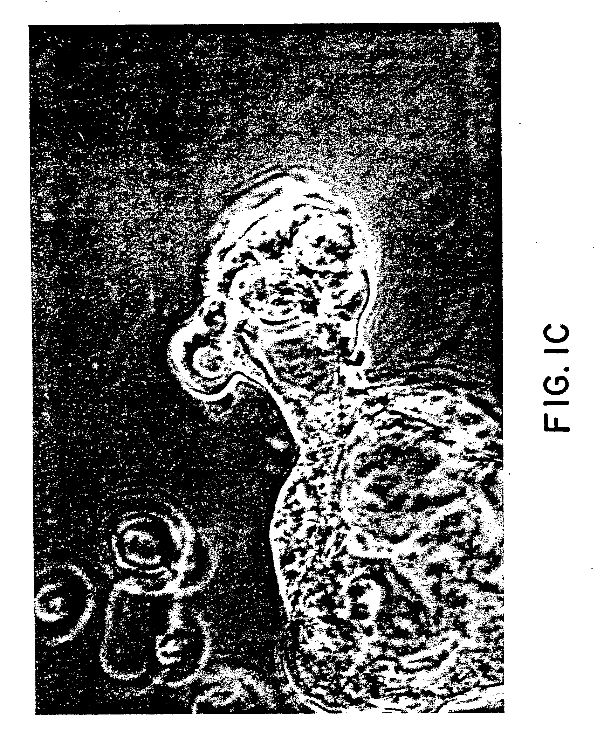

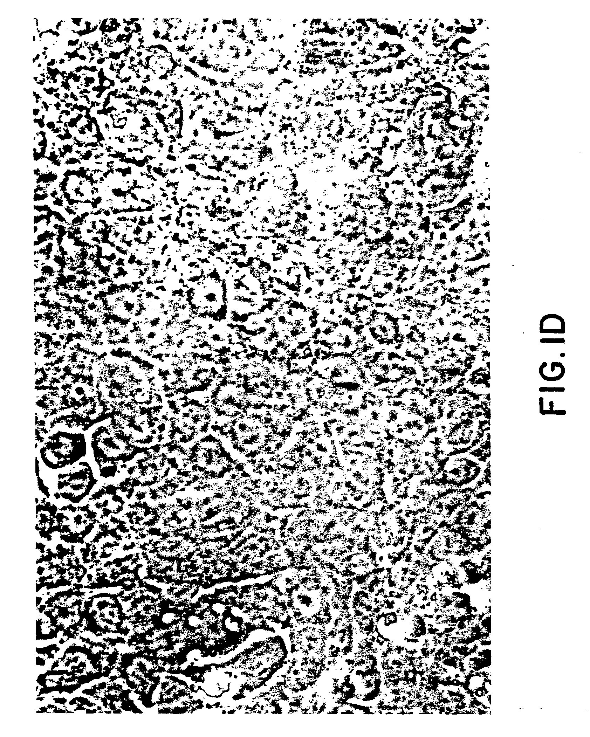

Figs. 1A, 1B, 1C and 1D depict phase contrast microscopy of cells of the NCI-H441-4, NCI-H820, NCI-H1404 and NCI-H322 cell lines, respectively.

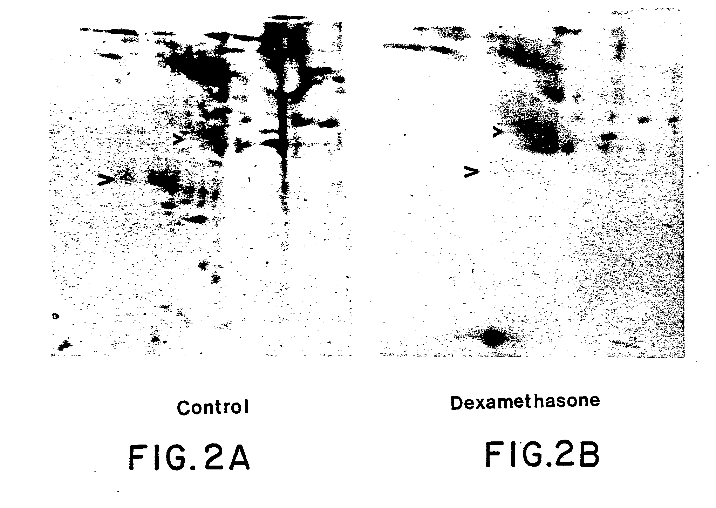

Figs. 2A and 2B depict [$^{35}$S]-methionine-labelling and 2D-IEF-SDS-PAGE (pH = 3.5-7.0) of the surfactant-associated proteins which are secreted into the media by NCI-H441-4 cells. The NCI-H441-4 cells are labelled with approximately 100 $\mu$Ci/ml of [$^{35}$S]-methionine. The NCI-H441-4 cells are labelled for approximately 6 hours, after which time the media is harvested with iced acetone (precipitated) and analyzed by IEF-SDS-PAGE. The NCI-H441-4 cells in Fig. 2A are cultured in the absence of dexamethasone whereas the NCI-H441-4 cells in Fig. 2B are treated for approximately 3 days with about 10 nM dexamethasone prior to labelling. Twice as much radio-label is applied in Fig. 2B to enable detection of SAP-35 which is inhibited by dexamethasone. In the control panel, Fig. 2A, the lower arrow marks the charge train typical of the SAP-35 protein and the upper arrow marks the small amount of the SPL(Phe) protein. In the presence of dexamethasone, the synthesis of the SAP-35 protein is markedly inhibited and several proteins induced, Fig. 2B. The upper arrow marks the SPL(Phe) protein which is induced over control levels, Figs. 2A and 2B. Several unknown proteins of smaller and larger molecular weights are also induced by dexamethasone as depicted therein.

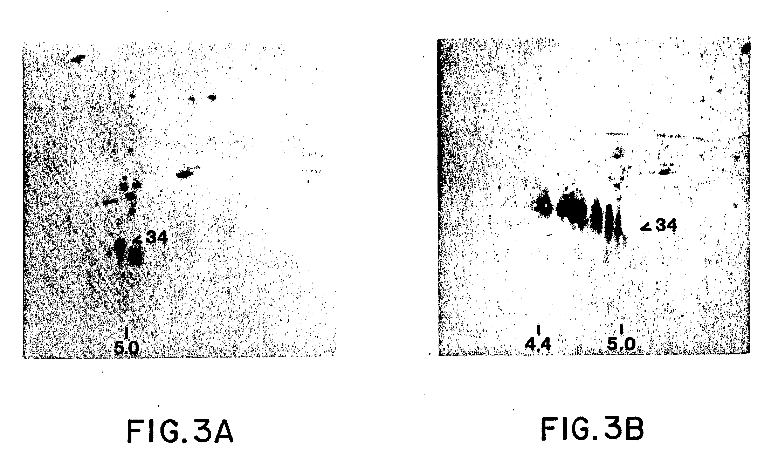

Figs. 3A and 3B depict the immunoprecipitation and purification of the SAP-35 protein from the cells and media of the cells of the NCI-H441-4 cell line after [$^{35}$S]-methionine-labelling and 2D-IEF-SDS-PAGE. Fig. 3A depicts an autoradiogram of the Mr = 30,000-35,000 SAP-35 intracellular precursor proteins marked < 34, pI approximately 5.0. These precursor proteins are indistinguishable in migration to those for the "high-mannose" precursors of the intracellular SAP-35 proteins previously detected in human lung explants. Fig. 3B shows the purification of the SAP-35 proteins from the media by immunoprecipitation. SAP-35 which is produced by NCI-H441-4 cells migrate with charge heterogeneity identical to that noted in the human alveolar lung lavage or amniotic fluid migrating with pI approximately 4.2 to 5.0 and Mr = approximately 30-35,000.

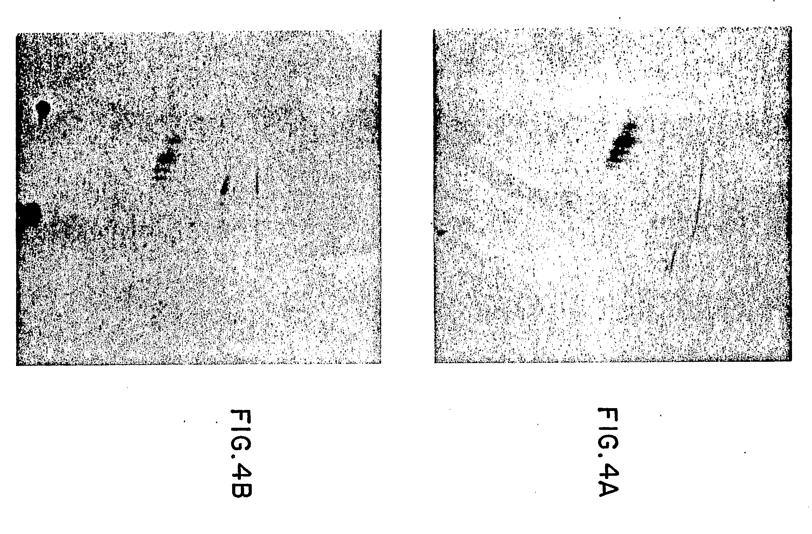

Fig. 4A represents affinity purification of [$^{35}$S]-methionine labelled SAP-35 from medium of NCI-H441-4 with an affinity column using a mouse monoclonal antibody generated against human SAP-35 conjugated to Sepharose 4B. Media from NCI-H441-4 containing [$^{35}$S]-methionine labelled SAP-35 is applied to the column after washing with a phosphate buffer, SAP-35 is eluted with 1M acetic acid and subjected to 2D-IEF-SDS-PAGE and autoradiography.

Fig. 4B represents affinity purification of [$^{35}$S]-methionine labelled SAP-35 from medium of NCI-H441-4 with an affinity column using a 30% NH$_4$SO$_4$ fraction of rabbit polyclonal antiserum generated against human SAP-35 conjugated to Sepharose 4B. Media from NCI-H441-4 containing [$^{35}$S]-methionine labelled SAP-35 is applied to the column; after washing with a phosphate buffer, SAP-35 is eluted with 1M acetic acid and subjected to 2D-IEF-SDS-PAGE.

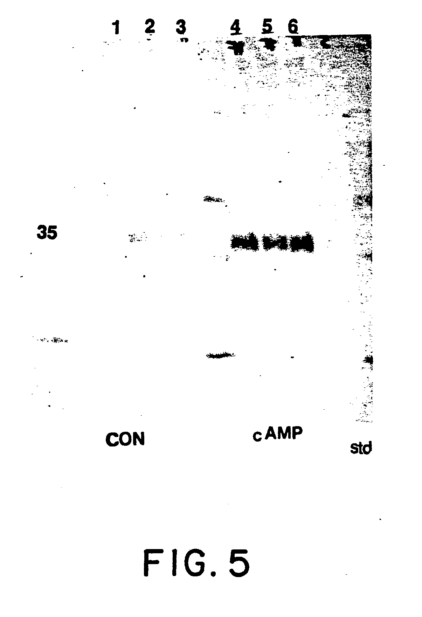

Fig. 5 depicts immunoprecipitation of the SAP-35 protein from NCI-H441-4 cells after [$^{35}$S]-methionine-labelling and 1D-SDS-PAGE. Lanes 1, 2, and 3 are triplicate cell cultures immunoprecipitated from untreated NCI-H441-4 cells, and lanes 4, 5, and 6 are treated with about 100 $\mu$M 8-Br-cAMP showing the induction of SAP-35 protein synthesis in the presence of 8 Br-cAMP. Induction of the SAP-35 proteins is observed within 24-48 hours.

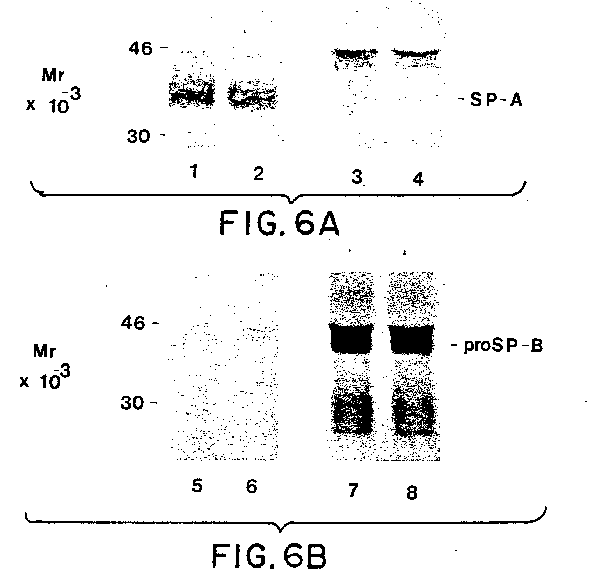

Figs. 6A and 6B depict the immunoprecipitation of the [$^{35}$S]-methionine labelled surfactant-associated proteins with and without 10 nM dexamethasone treatment. Control lanes 1-4 in Fig. 6A are immunoprecipitated with anti-SAP-35 serum after [$^{35}$S]-methionine labelling. The NCI-H441-4 cells are cultured in the absence (lanes 1 and 2) or presence of about 10 nM dexamethasone for 3 days (lanes 3 and 4 of Fig. 6A) which inhibits the production of the SAP-35 proteins marked SP-A. In Fig. 6B, a similar experiment is performed in which the NCI-H441-4 cells are cultured in the absence (lanes 1 and 2) or presence (lanes 3 and 4) of 10 nM dexamethasone for 3 days, precipitated with the anti-SPL(Phe) proteolipid antibody and demonstrating the marked induction of the 40-46,000 Dalton SPL(Phe) proteins (marked proSP-B) in response to dexamethasone. The appearance of bands at approximately $Mr = 27-33,000$ (also induced by dexamethasone) are likely to represent partial proteolytic processing of the proSPL(Phe) polypeptide. Presence of the SPL(Phe) protein precursors supports the presence of the proteolytic enzymes produced by the cell line for the proSPL-(Phe) under these culture conditions. The proteins are separated by SDS-PAGE, blotted to nitrocellulose and subjected to autoradiography for identical times.

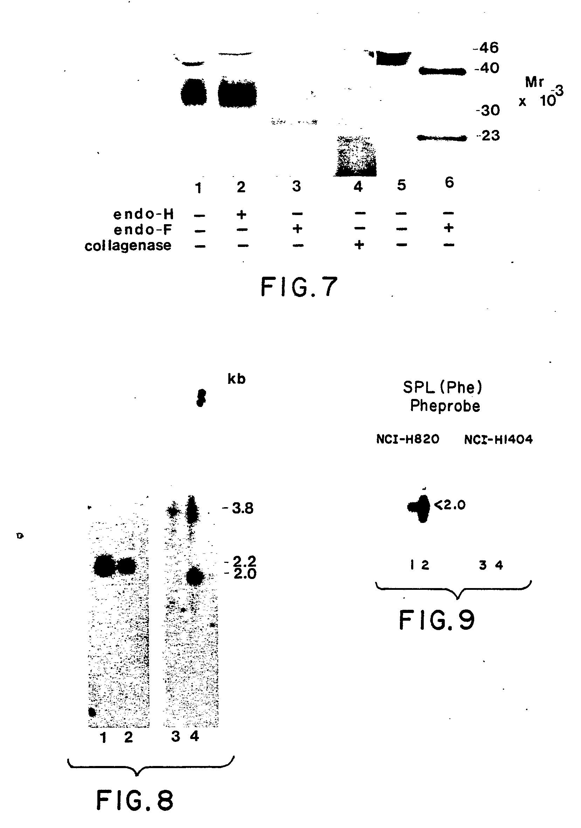

Fig. 7 depicts endoglycosidase and collagenase digestion of SAP-35 and pro SPL(Phe). The cells are incubated in the presence of 10nM dexamethasone (lanes 5 and 6) and absence of dexamethasone (lanes 1-4) for three days. Fig. 7 illustrates migration of control SAP-35 (lane 1) and the sensitivity of the SAP-35 proteins of the NCI-H441-4 cell line to endoglycosidase-H (lane 2-nonsensitive), endoglycosidase-F (lane 3-sensitive) and collagenase (lane 4-sensitive) illustrating the characteristic carbohydrate and peptide mapping of the SAP-35 proteins with these agents. Lanes 5 and 6 demonstrate immunoprecipitation of the SPL(Phe) proteins, $Mr = 40-46,000$ and $Mr = 27-33,000$, and the treatment of the SPL(Phe) proteins with endoglycosidase-F (lane 6-sensitive) which illustrates the fact that N-linked carbohydrate is present on the SPL(Phe) protein precursor which is removed by the addition of the endoglycosidase. The material migrating at 27-33,000 (fragments of 40-46,000 SPL(Phe) protein) is also sensitive to endoglycosidase-F migrating at approximately 23,000 after treatment with the endoglycosidase-F (lane 6). The [$^{35}$S]-methionine labelled proteins are immunoprecipitated from the NCI-H441-4 conditioned media prior to treatment with the enzymes and separated by SDS-PAGE followed by transfer to nitrocellulose and autoradiography.

Fig. 8 depicts Northern blot analysis of the mRNA encoding for the SAP-35 proteins (lanes 1 and 2) and the SPL(Phe) proteins (lanes 3 and 4) of the NCI-H441-4 cells. The cells in lanes 1 and 3 are cultured in the absence of dexamethasone whereas the cells in lanes 2 and 4 are cultured for 3 days in the presence of 10 nM dexamethasone. Approximately 12 µg of total mRNA are blotted to nitrocellulose and probed with a $^{32}$P-label- led cDNA specific for the SAP-35 (lanes 1 and 2) or SPL(Phe) (lanes 3 and 4). The size of the SAP-35 mRNA is about 2.15 to 2.2 kilobases, indistinguishable from normal human lung SAP-35 mRNA, Whitsett, J.A. et al: J. Biol. Chem., 262:5256-5261 (1987). The mRNA en- coding the SPL(Phe) of about 2.0 kilobases is indi- stinguishable from that recently demonstrated in nor- mal human lung. These studies identify the mRNAS en- coding the SAP-35 and SPL(Phe) of the NCI-H441-4 cell line and confirm the effects of dexamethasone on their expression.

Fig. 9 depicts a Northern blot analysis of the RNA encoding SPL(Phe) proteins produced by the cells of the NCI-H820 cell line in the presence and absence of about 10nM dexamethasone. RNA is extracted from control lanes 1 and 3 and subjected to Northern blot analysis with $^{32}$P cDNA encoding SPL(Phe). The NCI-H820 and NCI-H1404 cells are cultured in the absence (lanes 1 and 3) or presence of 10nM dexamethasone (lanes 2 and 4, respectively). Expression of mRNA encoding for SPL(Phe) is induced by dexamethasone in the NCI-H820 cell line as observed in lane 2. As to the expression of SPL(Phe) mRNA, it is not observed by the NCI-H1404 cell line in either the presence or the absence of dexamethasone (lanes 3 and 4).

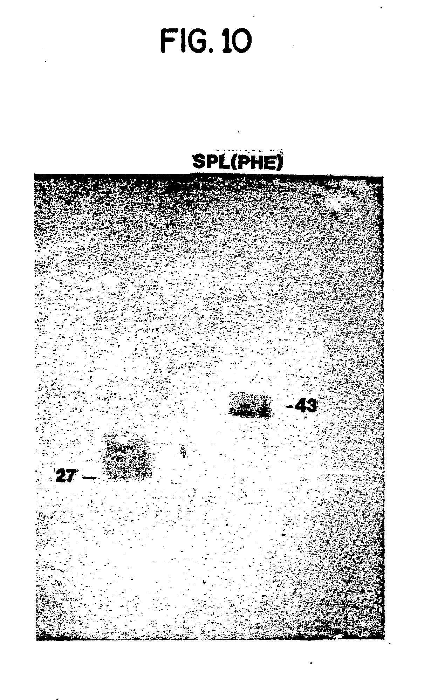

Fig. 10 depicts SDS-PAGE analysis of the purification of $Mr = 40-46,000$ protein (marked 43) in the right lane and $Mr = 27-33,000$ protein (marked 27) in the left lane, both of which are produced by and isolated from the NCI-H441-4 cell line. The dexamethasone treated cells are labelled with [$^{35}$S]-methionine, the media is collected, immunoprecipitated with anti-bovine surfactant proteolipid, analyzed by SDS-PAGE in about 13% polyacrylamide. The radio-labelled proteins are visualized by autoradiography. The proteins are excised from the wet gels, subjected to electroelution followed by SDS-PAGE, transferred to nitrocellulose and autoradiography.

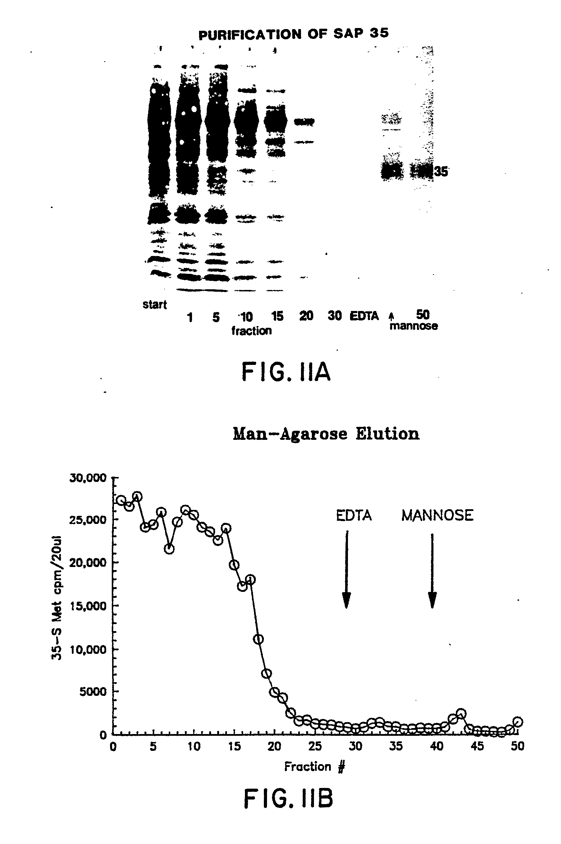

Fig. 11A depicts an autoradiogram of SAP-35 proteins which are produced by cells of the NCI-H441-4 cell line and purified with an α -methyl-mannose-agarose affinity column. Untreated NCI-H441-4 cells are labelled and the labelled media added to the columns. Purified SAP-35 is eluted with mannose (arrow).

Fig. 11B depicts a graphic illustration of purification of SAP-35 proteins which are produced by cells of the NCI-H441-4 cell line and purified with an α-methyl-mannose-agarose affinity column. As illustrated therein, the SAP-35 proteins are detected in approximately the first 3 mls of a mannose containing eluate.

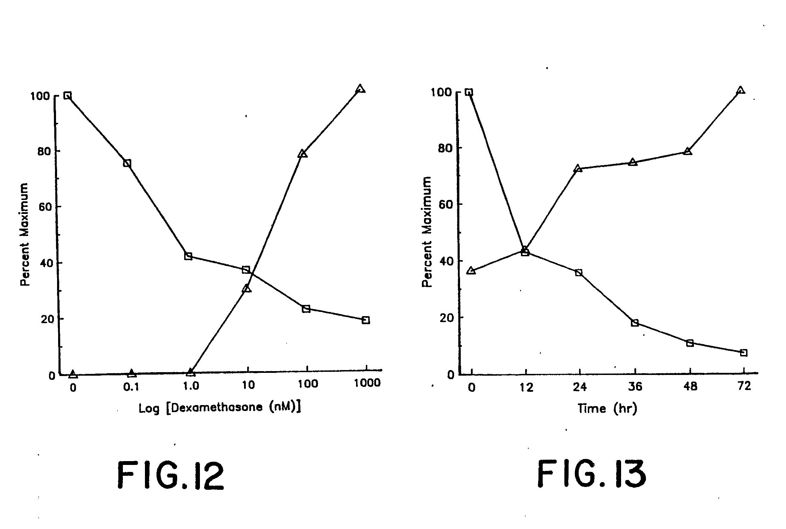

Fig. 12 depicts a dose response of dexamethasone on SAP-35 and ProSPL(Phe) synthesis. The cells of the NCI-H441-4 cell line are incubated in various concentrations of dexamethasone for 3 days prior to [$^{35}$S]-methionine-

labelling for 6 hours. SAP-35 (squares) and ProSPL(Phe) (triangles) are immunoprecipitated from media, separated by SDS-PAGE and electroblotted to nitrocellulose. Autoradiograms are then scanned densitometrically. Each point in Fig. 13 represents a scan of duplicate assays and is representative of two such experiments.

Fig. 13 depicts a time course of dexamethasone on SAP-35 and SPL(Phe) mRNA production. The cells of the NCI-H441-4 cell line are incubated in 10 nM dexamethasone for various times. Total mRNA is isolated as described herein and blotted to nitrocellulose. Nitrocellulose blots are probed with [$^{32}$P]-labelled SAP-35 (squares) and SPL(Phe) (triangles) cDNAs as described herein and the autoradiograms scanned. Quadruplicate dilutions of mRNA are scanned for each point in Fig. 14 to detect measurement in a linear range.

## Detailed Description of the Invention

By way of illustrating and providing a more complete appreciation of the present invention and many of the attendant advantages thereof, the following detailed description is given concerning the surfactant-associated proteins, continuous human cell lines, methods of producing and selectively regulating the production of at least one surfactant-associated protein and the particular mRNA encoding the protein, methods of purifying the surfactant-associated proteins, and methods to screen for tumor cells of pulmonary epithelial origin which produce surfactant-associated proteins.

## Surfactant-Associated Proteins

The terms "surfactant-associated protein" and/or "protein," are referred to herein interchangeably and in a broad sense and are meant to include any protein that is associated with the biophysical and biological properties necessary for pulmonary surfactant function and regulation. Exemplary of "surfactant-associated proteins" are SAP-35, SPL(Phe) and SPL(pVal). The terms "surfactant-associated protein" or "protein" are also intended to further include the preproteins, preproproteins, proproteins and any fragments thereof which are initially produced as precursors to the mature or fully processed surfactant-associated proteins. It is believed that the active surfactant-associated proteins are initially produced as preproproteins and proproteins which undergo complex post-translational processing to generate the fully processed or active surfactant-associated proteins. For example, it is discovered that the SPL(Phe) surfactant-associated protein is initially expressed by the NCI-H441-4 cell line as a novel Mr = 40-42,000 preproprotein which is then glycosylated at asparagine residues and sialylated resulting in a Mr = 40-46,000 proprotein, Figs. 6A, 6B, 7 and 10, and subsequently proteolytically cleaved to the Mr = 6-8,000 de-glycosylated and de-sialylated active or fully processed SPL(Phe) protein. It is further discovered that a novel protein of Mr = 27-33,000, also believed to be a precursor of SPL(Phe), is generated by the NCI-H441-4 cell line which is sensitive to endoglycosidase-F and neuraminidase digestion that results in an Mr = 23,000 de-glycosylated and de-sialyslated polypeptide, Figs. 6A, 6B, 7, and 10. The Mr = 23,000 Dalton protein has a pl of about 6.3-7.3. The Mr = 27-33,000 protein is believed to be a proteolytically cleaved fragment of the Mr = 40-46,000 SPL(Phe) precursor. The Mr = 40-46,000 and Mr = 27-33,000 proteins are relatively more soluble in water than SPL(Phe) and have pls of about 4.6-5.4 and 6.0-7.3, respectively. Since it is believed that the Mr = 40-46,000 and Mr = 27-33,000 proteins are rapidly proteolytically processed in vivo, it is unlikely that they can be extracted from human lung or alveolar lavage in significant amounts. With respect to the SPL(pVal) surfactant-associated protein, it is believed to be initially produced as an Mr = 22,000 precursor and subsequently processed to the active Mr = 5-6,000 protein found in the airway. Although the SPL(Phe) Mr = 23,000 precursor is similar in size to that of the SPL(pVal) Mr = 22,000 precursor, it is believed that the Mr = 23,000 protein is a precursor of SPL-(Phe) not SPL(pVal), since SPL(pVal) mRNA is not detected in the NCI-H441-4 cell line. The glycosylation pattern, immunoreactivity with antisera and migration after 1D-SDS-PAGE of the 23,000 Dalton protein are all consistent with its origin as a basic fragment of the Mr = 40-46,000 SPL(Phe) precursor, Fig. 7.

In addition to the novel human proteins isolated from the NCI-H441-4 cells and discussed herein, it is likely that other protein fragments of the 40-46,000 Dalton precursor are being generated by the NCI-H441-4 (Figs. 2A and 2B) and other human cells including for example, the fragment of the molecule cleaved to produce the 27-33,000 Dalton protein.

## Cell Lines

Novel cell lines which appear to produce at least one surfactant-associated protein have been established and designated as NCI-H441-4, NCI-H820, NCI-H1404 and NCI-H322, respectively. The cell lines have been established from human patients with lung adenocarcinoma. The cells of the cell lines are capable of growing in culture media for indefinite periods of time without undergoing significant transformation or loss of their differentiation or morphologic features. The morphological and cytochemical features of the adenocarcinoma cell lines are characteristic of Type II epithelial cells derived from human lung tissue, Figs. 1A, 1B, 1C and 1D. The NCI-H441-4, NCI-H820, NCI-H322 and NCI-H1404 cell lines all test positive for SAP-35 by ELISA type assay, and the NCI-H441-4 and NCI-H322 test positive for the SPL(Phe) synthesis by immunoprecipitation-autoradiography assays after $[^{35}S]$-methionine labelling as described herein confirming immunoreactivity with anti-surfactant proteolipid (directed primarily against SPL(Phe)). The NCI-H820 cell line produces SAP-35 as assessed by $[^{35}S]$-methionine labelling, immunoprecipitation-autoradiography assay. All four of the cell lines test negative for SPL(pVal) by Northern blot analysis.

The NCI-H441-4, NCI-H820 and NCI-H322 cell lines grow well in, for example, RPMI-1640 medium in about 10% fetal bovine serum and are readily passaged in continuous culture by gentle pipeting and dilution in new media. The preferred RPMI-1640 medium is the liquid (1C), form no. 320-1875, which is available through Gibco Laboratories and well known to those versed in this art. Alternative mediums, such as ACL-3 and ACL-4 mediums, may be employed to culture the cell lines as described in Gazdar, A.F. and Oie, H.B.: Letter to Editor, Cancer Research, 46:6011-6012 (November, 1986). Ascorbic acid may be continuously added to the medium in an amount of about 10-50mg/l to ensure adequate hydroxylation of the proline residues on SAP-35 protein. Vitamin K analogs may also be continuously incorporated into the medium in a sufficient amount to ensure adequate carboxylation of the glutamate residues on SAP-35. The cell lines tolerate extensive time in serum-free media and can be grown in serum-less media continuously. A variety of potentially useful serum-free media that may also be utilized for the cultivation of the cells are disclosed in Brower, M. et al: Growth of Cell Lines and Clinical Specimens of Human Non-Small Cell Lung Cancer in a Serum-free Defined Medium, Cancer Research, 46:798-806 (Feb. 1986). The cell lines are typically grown as monolayer cultures in serum containing media in about 95% air and about 5% $CO_2$ or in tightly-capped bottles in either tissue culture flasks, plates, petri dishes or roller bottles. Alternatively, such cultures can be grown on a variety of carrier beads with or without collagen or basement membrane proteins (Matrigel) designed for such purposes. All four tumor cell lines are readily passaged in culture by standard trypsinization or by gently pipeting the cells off the plates at approximately 1:3 dilution on a weekly or bi-weekly schedule. Serum-less defined media is highly useful for the purification of the proteins from the media.

## Methods for Regulating Expression

As already indicated above, the present invention contemplates a novel method for regulating the expression of a surfactant-associated protein by cells cultured in medium. Generally speaking, the method contemplates exposing cells cultured in medium to an effective regulating agent to regulate the expression of at least one surfactant-associated protein by the cells. This can be accomplished, for instance, by adding the regulating agent to the medium in which the cells are cultured. The term "regulating agent" is meant herein to include any effective agent that can be employed to induce and/or inhibit the production of at least one surfactant-associated protein and/or mRNA encoding the protein by cultured cells. Of course, it should be understood that for the regulating agent to have any effect on the cells to be regulated, the cultured cells must be capable of producing a surfactant-associated protein. Moreover, the cells must have the genetic capacity to regulate the production of such protein. Thus, in the case of cultured cells that have been genetically engineered, such cells typically will not be regulatable unless they are also genetically engineered for regulation. The present invention concerning regulation therefore envisions any cultured cells, naturally occurring or otherwise, that are capable of expressing a surfactant-associated protein and/or mRNA encoding the protein and regulating expression thereof.

The term "regulating agent" as used herein also contemplates the use of genes or viruses to transfect or infect, respectively, cultured cells to maintain them in a state of differentiation so that they will retain their ability to produce surfactant-associated proteins over long periods of time. Exemplary of such potential regulating agents include a variety of oncogenes such as ras, v-Ha-ras, epidermal growth factor-like oncogene such as erb B, and oncogenes such as N-myc, C-fos, etc. However, rather than adding a regulating agent of this type to the media to carry out exposure, exposure is accomplished by transfecting or infecting the cells with an effective gene or virus (the regulating agent), respectively, to alter de-differentiation and preserve expression.

For selectively regulating the expression of the SAP-35 proteins, the cells can be grown in the presence of a regulating agent such as 8-Bromo-derivative of cAMP (8-Br-cAMP) or other functional equivalents, Fig. 5. For selectively regulating the expression of SPL(Phe) proteins, the cells can be grown in the presence of a regulating agent such as a glucocorticoid hormone or other functional equivalents, Figs. 2A, 2B, 6A, 6B, 7 and 12.

The NCI-H441-4 cell line synthesizes SAP-35 without exposure to regulating agents. However, following regulation with 8-Br-cAMP, increased amounts of SAP-35 (Figs. 5 ) are observed in the tissue culture of the NCI-H441-4 cell line in which it secretes the SAP-35 proteins as a glycosylated, processed protein into the serum-free media. For instance, concentrations of SAP-35 of about 1-10 μg/ml have been found to accumulate in the media of near confluent NCI-H441-4 cells after several days in culture and can be increased by 8-Br-cAMP, as assessed by an ELISA type assay. Dexamethasone, however, dramatically enhances the expression of the SPL(Phe) precursors and SPL-(Phe) mRNAs allowing for selective manipulation of the production of this distinct protein and mRNA during culture, Figs. 2A, 2B, 6A, 6B, 7, 8, 12 and 13. Dexamethasone also selectively inhibits the synthesis of SAP-35 and SAP-35 mRNA, Figs. 2A, 2B, 6A, 6B, 7, 8, 12 and 13. For example, about 10 to about 100nM of dexamethasone in media inhibits SAP-35 protein and SAP-35 production in the NCI-H441-4 cell line on the order of about 2 to about 10 fold. In contrast, an increase in expression of the SPL(Phe) proteins assessed by metabolic labelling with [$^{35}$S]-methionine and in the mRNA level of the preprotein SPL(Phe) of more than about 2 fold is experienced by NCI-H441-4 cell line when dexamethasone is added to the medium in this concentration range. It should be understood that dexamethasone, like other regulating agents, induces the production of the SPL(Phe) proteins and inhibits the synthesis of SAP-35 in a dose dependent fashion, Fig. 12. Moreover, dexamethasone decreases the relative abundance of SAP-35 mRNA and increases the relative abundance of SPL(Phe) mRNA in a dose-dependent manner. The dose-dependent effects of dexamethasone on both SAP-35 and SPL(Phe) mRNA are similar to the dose-responses observed by [$^{35}$S]-methionine incorporation, Fig. 12 (not shown). The abundance of SAP-35 and SPL(Phe) mRNA is quantitated at various times after the addition of dexamethasone (10nM) which can be observed in Fig. 13. The inhibitory effects of dexamethasone on SAP-35 mRNA is detected at about 12 hours after the addition of the hormone with maximal inhibition occurring by approximately 48 hours. SPL(Phe) mRNA increases at about 12-24 hours after the addition of dexamethasone and remains increased even after 72 hours of exposure thereto, Fig. 13.

For dexamethasone, the EC$_{50}$ for inducing or inhibiting the NCI-H441-4 cell line is believed to be about 1-10nM. The SPL(Phe) proteins produced can be harvested from the cell lysates and/or conditioned media of the hormone-treated cells as described herein.

The NCI-H441-4 cell line is therefore unique in generating the surfactant-associated proteins known to be elaborated by Type II epithelial lung or Clara cells. Moreover, this cell line generates SAP-35 and SPL(Phe) in excessive and isolatable amounts when selectively induced. The NCI-H441-4 cell line therefore provides a means for the production of a fully processed or semi-processed surfactant-associated protein which is of physiologic interest. In addition, because of its ability to respond to regulation, the NCI-H441-4 cell line is also capable of expressing the mRNAs encoding the proteins in relatively large amounts. By employing conventional techniques, the mRNAs encoding the proteins may be separated from the mass of mRNAs present. Once isolated, the selected mRNAs can be used for production of cDNAs for these and other surfactant or pulmonary proteins of interest. It should be understood that sensitivity of the NCI-H441-4 cells or other cells employing the regulating methods of this invention may depend upon culture conditions, passage number, time of exposure to regulating agent, cell density, other components in the media and the like and therefore response may be variable.

Alternative regulating agents for regulating expression of surfactant-associated proteins and mRNA encoding therefore include, for instance, cAMP, cholera toxin, forskolin (about 10-100 μM), cAMP-phosphodiesterase inhibitors, such as isobutylmethylxanthine, theophylline, aminophylline, etc. (about 1-10mM), other derivatives of cAMP such as dibutyryl-cAMP (about 10-100 μM), phorbol esters such as tetradodeconyl 12-acetate-l3-myristate phorbolester (TPA) or phorbol-myristateacetate ester (PMA) (about 1-100 nM) and a variety of glucocorticoids, which are believed to be effective for the induction of SPL(Phe) preprotein and mRNA encoding therefore such as betamethasone, triamcinalone, hydrocortisone, cortisol or the like as well as fluorinated glucocorticoid agonists in amounts of about 0.1-100 nM. These types of regulating agents can be added to the media, preferably serum-free media, but alternatively dilute serum or other protein-containing media can be utilized. As to 8-Br-cAMP and dexamethasone, they can be added to the media to generate a concentration of about 10-100 μM and about 0.1-100 nM, respectively. It should be understood that the amounts and regulating agents provided above are intended to be exemplary only and not limitations and that any suitable amounts for these or other effective regulating agents are intended to be within the contemplation of this invention. Moreover, the amounts represent concentrations of the agents in the media.

In addition, the cell lines of the present invention may possibly be induced to produce other cellular proteins, such as proteases, anti-proteases and hormone induced proteins with the same or other regulating agents following the teachings of this invention, Figs. 2A and 2B.

## Methods for Purifying Protein

Purifying protein from cell lysates or media can be accomplished after selectively regulating the expression of a specific protein of interest. SPL-(Phe) proteins can be selectively induced as discussed above by culturing the cells in medium containing, for example, glucocorticoids or other suitable hormones alone or in combination. Hormones can be added for about 24-72 hours after which time the media can be collected. The SAP-35, $Mr = 30-35,000$, and SPL(Phe) precursors, $Mr = 40-46,000$, $Mr = 27-33,000$ and $Mr = 23,000$, can be harvested after continuous exposure or after short-term exposure from cells or medium. Culture periods can be as short as 24 hours or as long as a week or more during continuous culture. Following induction, the media and/or cells will be highly enriched for the protein of interest. The cells can be maintained in the hormone containing media for continuous removal of the protein of interest for purification.

SAP-35 proteins can be readily purified from cell lysates or media by a number of techniques which include, for example, isoelectric focusing using methods as described by Ross, G.F. et al: Structure of the Major Glycoproteins from Human Alveolar Proteinosis Surfactant, Biochim. Biphys. Acta., 911:294 (1987), fast protein liquid chromatography (FPLC) obtained from Pharmacia using isoelectric focusing followed by molecular seive chromatography, and Cibacron Blue affinity chromatography. Affinity purification using monoclonal and polyclonal antibodies or alpha-methyl-mannose coupled to a sepharose 4B or agarose column is useful for SAP-35 purification as depicted and described in Figs. 4A, 4B, 11A and 11B herein. Standard technology can be used to conjugate the antibodies to the affinity column. Following application of the cell lysates or medium to the column, the proteins can be eluted with, for example a hapten sugar, such as mannose for the $\alpha$-methyl-mannose agarose columns with EDTA, EGTA. Highly concentrated salts, highly acidic or basic buffers, such as 1M acetic acid, can be used to elute the protein from immunoaffinity columns such as described herein. In addition, phospholipid vesicles may be added to the media to bind with the SAP-35 proteins to selectively remove them from other proteins prior to purification.

Purification of SPL(Phe) precursor proteins can be accomplished with immunoprecipitation followed by SDS-PAGE and electroelution, Figs. 6A, 6B, 7 and 10. In addition to this method, there are likely to be alternative methods for purification of the SPL(Phe) precursor proteins. For example, cell lysates or media can be collected and subjected to DEAE-cellulose resin or to isoelectric focusing since the $Mr = 40-46,000$ preprotein has an acidic pI of about 4.6-5.4. Purification may be performed in the presence or absence of ionic or non-ionic detergents, such as NP-40 and sodium dodecyl sulfate. Lectin affinity and molecular seiving chromatography and affinity chromatography using polyclonal or monoclonal antibody generated against SPL(Phe) or its precursor may be used for purification. As demonstrated with anti-SAP-35, standard technology can be used to conjugate the SPL(Phe) antibodies to the column. SPL(Phe) fragments are more hydrophobic than the precursors and associate with phospholipids, such as phosphatidylcholine and phosphotidylglycerol, which may be used to also purify the SPL(Phe) proteins or its precursor peptides as phospholipid-protein complexes which can be purified by ultracentrifugation using buoyant density as used for lipoproteins. It may be also useful to treat the SPL-(Phe) proproteins with one or more proteases to produce the small hydrophobic peptides prior to purification. Various hydrophobic fragments of the SPL(Phe) precursors can be purified by, for example, chloroform/methanol or n-butanol or ether/ethanol solvent extraction as has been utilized and described heretofore to isolate the hydrophobic proteins, e.g., Whitsett, J.A. et al: Pediatr. Res., 20:460-467 (1986) and Whitsett, J.A. et al: Pediatr. Res., 20:744-749 (1986).

As indicated above, the NCI-H441-4 cell line produces a $Mr = 27-33,000$ Dalton protein, likely a proteolytic fragment of the $Mr = 40-46,000$ proSPL-(Phe), which can be purified using similar techniques discussed above. The production of proteolytic fragments of SPL(Phe) also suggests that the NCI-H441-4 cell line may be a source of the proteases which are required for the proteolytic processing of SPL(Phe). The SPL(Phe) precursors can be further purified using antibody generated against SPL(Phe) by immunoprecipitation or immunoaffinity chromatography to provide complete and rapid purification. An example of $Mr = 40-46,000$ and $Mr = 27-33,000$ SPL(Phe) precursor purified by immunoprecipitation are seen in Figs. 6A, 6B, 7 and 10. Medium from the NCI-H441-4 cell line is first radio-labelled with [$^{35}$S]-methionine after dexamethasone exposure and precipitated with polyclonal antisera recognizing human SPL(Phe) which was generated against bovine hydrophobic proteolipid proteins. The SPL(Phe) peptides are then subjected to NEPHGE-SDS-PAGE analysis to identify the SPL(Phe) proteins. Since some of the SPL(Phe) precursors are glycosylated in the NCI-H441-4 cell line, the purification can also be enhanced utilizing a number of selective lectin affinity chromatography agents such as concanavalin A-sepharose, wheat germ agglutinen-sepharose (WGA), etc. For analysis and purification of the Mr = 27-33,000 proteins, NEPHGE-SDS-PAGE is preferred since this protein does not readily enter conventional 2D-IEF-SDS-PAGE, P.Z. O'Farrell et al: High Resolution Two-Dimensional Electrophoresis of Basic as Well as Acidic Proteins, Cell, 12:1133-1142 (December, 1977), which is incorporated herein in its entirety by reference. However, all identified SPL(Phe) precursors Mr = 40-46,000, Mr = 27-33,000 and Mr = 23,000 can be purified on 1D-SDS-PAGE as seen in Fig. 7. For analysis and purification of the Mr = 23,000 proteins, 1D-SDS-PAGE or NEPHGE-SDS-PAGE can be utilized and they can be purified using the identical methods described for purifying the Mr = 27-33,000 proteins, Fig. 7.

Furthermore, as already indicated, the addition of phospholipids may be used to selectively bind the lipid binding proteins and separate those from other non-lipid binding proteins secreted by these cells with then further purification by sizing or affinity chromatography. Albumin can be removed by using Cibacron blue chromatography or other albumin binding affinity resins which will remove contaminating serum albumin. The identification of the proteins can be further accomplished by co-migrating the synthesized protein with known protein markers and treatment of the proteins with endoglycosidase-H or endoglycosidase-F to demonstrate the presence of N-linked glycosylation and their co-migration with the proteins present in the airway or synthesized by normal lung tissue. Proteolytic processing of the SPL(Phe) proteolipid precursors of Mr = 40-46,000 to a precursor of Mr = 27-33,000 occurs at a significant rate in tissue culture. Although complete processing to the hydrophobic Mr = 6-8,000 SPL(Phe) is observed following addition of the NCI-H441-4 medium to rat Type II cells, SPL(Phe) itself has not as of yet been isolated from the cell lines described herein.

The purified precursor proteins may be treated with appropriate lung proteases, metalloproteases, elastase, trypsin, papain, staph V-8 protease, thermolysin, cathepsins, chymotrypsin or other standard purified proteases for the generation of the appropriate fragments which may be useful for specific therapies. Since the hydrophobic proteins, SPL(Phe) and SPL(pVal), are resistant to protease digestion, Whitsett, J.A. et al: Pediatr. Res., 20: 460-467 (1986), the N- and C-terminal residues can be removed from the precursor proteins resulting in the fully processed or matured form of the "active" surfactant peptides.

Though SPL(pVal) is not expressed in detectable abundance by NCI-H441-4 cell, it is likely that similar procedures will be useful for the identification and induction of protein expression for SPL-(pVal) or other important Type II cell proteins in other cells or similar cell lines. Lectin binding, however may not be helpful since N-linked carbohydrate has not been detected in SPL(Val) and the predicted amino acid sequence derived from the cDNAs predicts a lack of N-linked carbohydrate.

Methods for Screening Cells

To screen for tumor cells of pulmonary epithelial origin that produce surfactant-associated proteins, the cells should be first grown in standard culture media such as in RPMI-1640, ALC-3 or ALC-4 media containing 5-10% fetal bovine serum in 95% air and 5% $CO_2$ on plasticware. Tissue culture flasks can then be plated therefrom at densities such that cells reached near confluence prior to the experiments. The cells should then be scraped from the plates and media washed away. Lysates can now be prepared therefrom by sonication in dilute buffer with or without NP-40 or other detergent. The lysates should then be tested in an ELISA type assay for SAP-35. The ELISA type assay may consist of purified goat anti-human SAP-35 which is purified by ammonium sulfate fractionation and applied to the plate. The lysate is added to the plate followed by the addition of second antibody rabbit anti-human SAP-35 followed by a third anti-rabbit conjugate antibody containing immunoperoxidase which is then developed by standard O-phenylaminediamine reaction to generate color. If the cells test positive for the SAP-35 by the ELISA type assay, they should be further studied to optimize conditions for surfactant-associated protein expression and to optimize conditions for their characterization, synthesis and production. To accomplish this, the screened cells should be grown as above and cultured in the presence or absence of for example, 100 μM 8-Br-cAMP and/or 10 nM dexamethasone or the like which is added to the culture approximately 24-72 hours prior to the planned experiments. After this time, the cell media should be washed away and replaced with methionine deficient serum-free me-

dia. [$^{35}$S]-methionine is then added to the newly replaced media to generate a media concentration of about 100 μCi/ml for 4-6 hours following which the media is harvested and subjected to immunoprecipitation with anti-SAP-35 or anti-surfactant proteolipid (SPL(Phe) and SPL(pVal)) antiserum or antibody. The material can then be subjected to either 1D-SDS-PAGE or 2D-IEF-PAGE and autoradiogramed. Media can also be collected for 2D-SDS-PAGE before or after immunoprecipitation.

Alternatively, the labelled proteins can be separated, by 1D-SDS-PAGE, NEPHGE-SDS-PAGE or 2D-IEF-SDS-PAGE, transblotted to nitrocellulose, dried and applied to autoradiography and immunoblot analysis for identification of the proteins. Total proteins which are labelled with [$^{35}$S]-methionine or other radio-labelled amino acids can be harvested and precipitated in trichloroacetic acid and counted to assess the percentage of total protein secreted to assess the percent secreted of the surfactant-associated proteins. The cells can be homogenized by mechanical homogenization, lysis or nitrogen cavitation followed by differential centrifugation to enrich cell fractions with SAP-35 or SPL(Phe) or SPL(pVal). Media or cell lysates can be used directly for immunopurification. Immunoprecipitation can be performed using protein A-sepharose to bring down the immunoconjugate.

Cells can also be used for mRNA isolation by standard methodology of Chirgwin et al: Biochem., 18:5294-5299 (1979). Cells should be cultured however till near confluence, harvested and total RNA prepared after homogenization as described by Chirgwin. The RNA can then be utilized for Northern blot analysis after separation on agarose gels and transblotting to nitrocellulose which is then utilized for hybridization analysis with cDNA clones which are radio-labelled with $^{32}$P for identification of the specific messenger RNAs in the tumor lines under various conditions.

It should be appreciated that even though the cell lines described herein have not screened positive for SPL(pVal), the methods described herein can be used to screen for other cell lines which might express SPL(pVal) or other proteins. In addition, immunofluorescence using anti-SPL(Phe) or anti-SAP-35 antisera and subsequent fluorescence microscopy can be used to screen cell lines. Similarly, radioimmunoassay or dot blot assay could be utilized to screen for surfactant protein production.

### Techniques

Most techniques which have been used to generate the antibodies and cDNA probes, to isolate proteins from normal tissue, to identify the proteins expressed by the cell lines and the like are widely practiced in the art, and most practicioners are familiar with the standard resource materials which describe specific conditions and procedures. However, for convenience the following paragraphs and references identified therein, which are incorporated in their entireties herein by reference, may serve as guidelines.

Moreover, the invention and techniques embodied herein are described in O'Reilly, M.A. et al: Differential Effects of Glucocorticoid on Expression of Surfactant Proteins in a Human Lung Adenocarcinoma Cell Line, submitted to J. Biol. Chem. for publication, (1987) which is filed contemporaneously herewith and incorporated herein in its entirety by reference.

### Purification of Human Proteolipid Surfactant-Associated Proteins

Lung surfactant is purified by differential centrifugation and the hydrophobic proteins extracted by chloroform/methanol or ether/ethanol extraction, Whitsett, J.A. et al: Pediatr. Res., 20:460-467 (1986) and Whitsett, J.A. et al: Pediatr. Res., 20:744-749 (1986). Protocols for use of human tissue are approved by the Human Research Committee, University of Cincinnati College of Medicine. Human preparations contain two surfactant-associated apoproteins, SPL(Phe) and SPL(pVal), identified by their distinct N-terminal amino acid sequences, Glasser S.W. et al: Proc. Natl. Acad. Sci., USA, 84:4007-4011 (1987) and Glasser, S.W. et al: cDNA, Deduced Polypeptide Structure and Chromosomal Assignment of Human Pulmonary Surfactant Proteolipid: SPL(pVal), submitted to J. Biol. Chem. for publication (1987) and filed contemporaneously herewith. The N-terminal sequence of SPL(Phe) peptide Phe-Pro-Ile-Pro-Leu-Pro-Tyr, etc., represents approximately 2/3 of the sequence obtained in the human preparation as previously reported, Glasser, S.W. et al: Proc. Natl. Acad. Sci., USA, 84:4007-4011 (1987). The remainder consists of SPL(pVal): Ile-Pro-Cys-Cys-Pro-Val-His-Leu, etc. (exact amino acids derived from the cDNA and gene sequence). The nucleotide and amino acid sequences described in Glasser, S.W. et al: Proc. Natl. Acad. Sci., USA, 84:4007-4011 (1987) are specifically incorporated herein by reference.

### Purification of Human SAP-35

Surfactant is purified from lung lavage fluid from human volunteers and patients with alveolar proteinosis, from amniotic fluid obtained at term Cesarean section and from lung lavage of human cadavers at autopsy. All protocols are approved by the Human Research Committee, University of Cincinnati College of Medicine. Human surfactant is purified by differential centrifugation and SAP-35 from lung lavage is purified to homogeneity by preparative isoelectric focusing and Cibacron-blue affinity chromatography as described in Ross, G.F. et al: Biochim. Biophys. Acta., 870:267 (1986).

## Preparation of Polyclonal Antiproteolipid Antibody

Antiserum (polyclonal antibody) is generated in rabbits by repeated injection of bovine surfactant-associated proteolipid prepared by chloroform/methanol extraction of surfactant, Whitsett, J.A. et al: Pediatr. Res., 20:460-467 (1986) and Whitsett, J.A. et al: Pediatr. Res., 20:744-749 (1986). This antiserum (bovine surfactant proteolipid antiserum) immunoprecipitates both the 40-46,000 Dalton SPL(Phe) and the 22,000 Dalton SPL(pVal) precursors, Glasser, S.W. et al: Proc. Natl. Acad. Sci., USA, 84:4007-4011 (1987); Glasser, S.W. et al: cDNA, Deduced Polypeptide Structure and Chromosomal Assignment of Human Pulmonary Surfactant Proteolipid: SPL(pVal), submitted to J. Biol. Chem. for publication, 1987; and Whitsett et al: Glucocorticoid Enhances Surfactant Proteolipid SPL(Phe) and SPL(pVal) Synthesis and RNA in Fetal Lung, submitted to J. Biol. Chem. for publication, 1987 and filed contemporaneously herewith. The antiserum reacts with protein of Mr = 5-14,000 (in presence of $\beta$-mercaptoethanol) in immunoblots of organic extracts of bovine, human and canine surfactants, Whitsett, J.A. et al: Pediatr. Res., 20:460-467 (1986) and Whitsett, J.A. et al: Pediatr. Res., 20:744-749 (1986).

## Preparation of Polyclonal SAP-35 Antibody

SAP-35 is purified to homogeneity, using surfactant obtained from patients with alveolar proteinosis, Ross, G.F. et al: Purification of Canine Surfactant-Associated Glycoproteins A. Identification of a Collagenase-Resistant Domain, Biochim. Biophys. Acta., 870:267 (1986). Antisera (polyclonal antibodies) are prepared by repeated injections of approximately 300 micrograms of purified protein in Freund's complete adjuvant into goats and New Zealand albino rabbits, Weaver, T.E. et al: Synthesis of Surfactant-Associated Protein-35,000 in Fetal Lung Organ Culture, J. Appl. Physiol., 61:694 (1986). Specificity of the antisera for SAP-35 and its oligomers is determined by immunoblot analysis of normal SAP-35 obtained from amniotic fluid or alveolar lavage of alveolar proteinosis surfactant. Characteristics of the antisera are reported, Weaver, T.E. et al: Synthesis of Surfactant-Associated Protein-35000 in Fetal Lung Organ Culture, J. Appl. Physiol., 61:694 (1986).

## Enzymes

Endoglycosidase-H and -F can be obtained from New England Nuclear (Boston, MA). Protein samples are immunoprecipitated as described herein. For endoglycosidase-H digests, pellets are resuspended in 250 $\mu$l of 50 mM sodium acetate pH = 5.5 and 0.1% Nonidet P-40 (LKB). Enzyme is added to a final concentration of 1 $\mu$g/ml. For endoglycosidase-F digests, immunoprecipitates are resuspended in 50 mM sodium phosphate pH = 6.1, 5 mM EDTA and 0.1% Nonidet P40. Enzyme are added to a final concentration of 2 U/ml. For collagenase digests, immunoprecipitates are resuspended in 100 mM Tris-HCl pH = 7.3, 100 mM NaCl, 0.5 mM MgSO$_4$, 0.5 mM CaCl$_2$ and 0.1% Nonidet P-40. Collagenase is added to a final concentration of 100 U/ml. All enzyme digests are incubated at 37°C with constant shaking for 18 hours.

## Proteolipid Surfactant-Associated ELISA Type Assay

ELISA of the surfactant proteolipid is performed using the anti-proteolipid antibody which recognizes both SPL(Phe) and SPL(pVal) and as prepared herein, Whitsett et al: Glucocorticoid Enhances Surfactant Proteolipid SPL(Phe) and SPL-(pVal) Synthesis and RNA in Fetal Lung, submitted to J. Biol. Chem. for publication, 1987. Competition curves are generated with purified bovine proteolipids containing predominantly SPL(pVal), or mixtures of the two proteins SPL(Phe) and SPL-(pVal). The standards completely inhibit immunoreactivity of the antibody under assay conditions. Standard proteolipid protein is estimated after silver staining and/or in relationship to previous amino acid analysis after hydrolysis in HCL, Glasser, S.W. et al: Proc. Natl. Acad. Sci., USA, 84:4007-4011 (1987). This antiserum is reactive with both proteolipid precursors in immunoprecipitation assays. ELISA is performed by competition assay using purified, predominantly delipidated proteolipids which are absorbed to each of the plastic wells. The proteolipid preparation inhibits the reactivity of the antiserum in a dose dependent manner (10-100 ng). Assays of the cell homogenates can be performed at two or three different dilutions of the sample in 10% propanol in water to assure parallel dilution in the competition curve. Cell samples can be homogenized immediately or frozen at -30° C for 1-2 weeks prior to homogenization in phosphate-buffered saline, pH 7.2, containing 10 mM EDTA and 1mM PMSF. Sample and anti-bovine proteolipid PMSF antiserum (1:30,000 or other similar dilutions) are preincubated overnight at 37° C prior to addition to the well. The reaction is then developed with immunoperoxidase conjugated goat anti-rabbit IgG (1:1000) and 0-phenylenediamine.

## SAP-35 ELISA Type Assay

SAP-35 concentration is determined by an ELISA (enzyme-linked capture immunoassay) type assay utilizing goat and rabbit antisera prepared as described herein. The assay is performed in Falcon microtiter plates (Becton Dickinson and Co., Oxnard, CA). Standard curves are constructed using human SAP-35 purified to homogeneity from patients with alveolar proteinosis as described. Composition, detailed tryptic peptide mapping by high performance liquid chromatography and thin layer chromatography of the alveolar proteinosis protein as reported in Ross, G.F. et al: Structure of the Major Glycoproteins from Human Alveolar Proteinosis Surfactant, Biochim. Biophys. Acta., 911:294 (1987). The protein standard is quantitated by the method, Lowry, O.H. et al: Protein Measurement With the Folin-Phenol Reagent, J. Biol. Chem., 193:265 (1951), using bovine serum albumin as control after hydrolysis and amino acid determination. Plates are coated overnight at 4° C with 1:100 goat anti-human SAP-35. The wells are then washed extensively with "washing buffer" consisting of 0.01M Tris HCl (pH 8.0) and 0.05% Tween. Non-specific protein binding to wells "blocked" with buffer containing 150 mM NaCl, 10 mM Tris-HCl (pH 7.4), 5 mg/ml bovine serum albumin, 5% goat and 5% human serum. The wells are dried, followed by addition of the amniotic fluid samples. SAP-35 containing fluid, media or cell lysates is diluted from 1:50 to 1:800 in "dilution buffer" containing 150 mM NaCl, 50 mM Na₂HPO₄PO₄ (pH 7.4) with 0.5% NP-40. Samples to be assayed (100 μl) are added to the wells (in triplicate) and incubated for two hours at 37° C. The wells are again washed extensively with "washing buffer" followed by addition of rabbit antihuman SAP-35. Rabbit antisera is diluted 1:2000 in "blocking buffer" (containing 5% human and 5% goat serum) and incubated for one hour at 37° C. Wells are washed with washing buffer and then a third antibody, goat anti-rabbit immunoglobulin, which is conjugated to horseradish peroxidase (Calbiochem, LaJolla, CA) is added at a 1:3000 dilution. This antibody is diluted in "dilution buffer" with 0.05% (v/v) Tween 20 in 5% goat and 5% human serum. Plates are incubated for one hour at 37° C followed by repeated washes with "washing buffer". Substrate (0.2% 0-phenylenediamine and 0.015% H₂O₂) in 17 mM citric acid and 65 mM Na₂HPO₄/NaH₂PO (pH 6.3) is added to the wells at room temperature. The reaction is stopped after 30 minutes with 100 μl of 5M sulfuric acid and the absorbance is read at 490 nm. The assay performed in this fashion had an interassay coefficient of variation of approximately 5%. SAP-35 is stable during storage. Chemicals used in making the reagents and buffers are obtained from SIGMA Chemical Company, St. Louis, MO. The cell lines are screened for production of SAP-35 using the procedures identified above.

## Isolation of Surfactant-Associated Proteins for Gel Analysis

Cells are grown to near confluence in RPMI-1640 medium (GIBCO) containing 10% fetal calf serum. Dexamethasone (100nM), 8-Br-cAMP (100 μM) (Sigma, Inc., St. Louis, MO) are added to stated concentrations for approximately 48 to 72 hours. Media is changed to serum-free, methionine-deficient media for labelling. Metabolic labelling of cells is performed in methionine-deficient medium (1 mg/1 unlabelled methionine) in serum-free medium. [³⁵S]-methionine (New England Nuclear, Boston, MA), specific activity = 1000 Ci/mmol, is added to a final concentration of 100-150 μCi/ml and the cells are incubated for approximately 5-6 hours. [³⁵S]-methionine-labelled cells are prepared for electrophoretic analysis after sonication in 190 mM NaCl, 6 mM EDTA, 4% SDS, 1 mM phenylmethylsulfonylfluoride and 68 mM Tris-HCl, pH 7.4. Duplicate aliquots of the sonicate are precipitated with trichloracetic acid for determination of [³⁵S]-methionine incorporation. Media is collected by cold acetone precipitation and placed in sample buffer for SDS-PAGE and NEPHGE-SDS-PAGE analysis of [³⁵S]-labelled pro teins.

## Gel Analysis of Surfactant-Associated Proteins

Protein samples are analyzed under reducing conditions by sodium dodecylsulfate-polyacrylamide gel (SDS-PAGE) electrophoresis (13%) or by two-dimensional isoelectric focusing polyacrylamide gel (2D-IEF-SDS-PAGE) electrophoresis (13%) (pH 3.5-5.8), Garrison, J.C. et al: J. Biol. Chem., 257:13135-13143 (1982). Proteins are electrophoretically transferred to nitrocellulose which is subsequently dipped in 20% 2,5-diphenyloxazole in toluene and exposed to Kodak XAR-2 film at -70°C or identified by direct autoradiography after SDS-PAGE before or after transfer to nitrocellulose. 1D-SDS-PAGE or NEPHGE-SDS analysis is required to identify the Mr = 27-33,000 fragment of the Mr = 40-46,000 precursor of SPL(Phe) using similar technology. Immunoblot or silver staining is utilized to detect the protein after gel analysis as described in Sammons et al: Electrophoresis, 2:135-141 (1981) and Whitsett et al: Biochimica Biophys. Acta., 828:162-171 (1985).

## mRNA Extraction for Hybridization

Cells are homogenized in buffer containing 4M guinidine thiocyanate, 0.5% N-lauroyl sarcosine, 20 mM sodium citrate 0.1M β-mercaptoethanol and 0.1% antifoam A. RNA is extracted either by precipitation in lithium chloride, Cathala, G. et al: DNA, 2:329-335 (1983), or by centrifugation through a cushion of 5.7M cesium chloride, Chirgwin, J.M. et al: Biochem., 18:5294-5299 (1979). The RNA pellet is dissolved in solubilizing buffer containing 0.1% SDS, 1 mM EDTA, 10 mM Tris-HCl, pH 7.5 or in water, extracted with phenol and chloroform, and precipitated with ethanol. The amount of RNA in an aqueous solution is determined by optical density at 260 nm. In order to minimize variability among samples, control and treated cells are processed and analyzed in parallel.

## Northern Blot and Slot Blot

Nitrocellulose filters containing mRNA are hybridized with a 1.5 Kb SPL(Phe) cDNA clone (214.1), a 0.3 Kb SPL(pVal) cDNA clone (334.2), or a 0.9 Kb SAP-35 cDNA clone (7.1) isolated from a λgtll expression library generated from adult human lung poly A + RNA: Glasser, S.W. et al: Proc. Natl. Acad. Sci., USA, 84:4007-4011 (June, 1987); Glasser, S.W. et al: cDNA, Deduced Polypeptide Structure and Chromosome Assignment of Human Pulmonary Surfactant Proteolipid: SPL(pVal), submitted to J. Biol. Chem. for publication (1987); Whitsett, J.A. et al: J. Biol. Chem., 262:5256-5261 (1987); and Whitsett, J.A. et al: J. Biol. Chem.,

262:7908-7913 (1987). The SAP-35 protein clone is ligated in pUC18, and the cDNA consists of nearly the entire coding sequence of SAP-35. The SAP-35 protein clone begins at the seventh base into the coding sequence and extends 100 base pairs into the 3' untranslated region. The SAP-35 cDNA probes are labelled with [ α-$^{32}$P]-dCTP using a random primer labelling kit (Pharmacia) or a nick translation reagent kit (Bethesda Research Laboratories). In some experiments, filters are washed free of SPL(Phe) probe and reprobed in an identical fashion with the SAP-35 cDNA which contains nearly the entire coding region of SAP-35, Whitsett, J.A. et al: J. Biol. Chem., 267:7908-7913, (1987) and Whitsett, J.A. et al: J. Biol. Chem., 262:5256-5261, (1987).

For Northern blot analysis, mRNA can be prepared from cells and is separated on a 1.2% agarose formaldehyde gel, Maniatis, T. et al: Molecular Cloning, A Laboratory Manual. Cold Spring Harbor Laboratory, Cold Spring Harbor, New York, pp. 125-126 (1982), and transferred to nitrocellulose. The filter can be baked at 80°C for 2 hours. Nitrocellulose "slot" blots can be made by applying approximately 250-500 ng of formaldehyde-denatured RNA to nitrocellulose using a "Slot" blot manifold (Schlicher and Schuell, Inc.). For Northern blot analysis, 5-12 μg RNA is applied. The filters can then be prehybridized in 40% deionized formamide, 5XSSC (1 x SSC is 0.15 M NaCl, 0.015 M sodium citrate) 50 mM NaH$_2$PO$_4$, pH 7.0, 5 x Denhardt's solution (1 x Denhardt's is 0.02% bovine serum albumin, 0.02% Ficoll, 0.02% polyvinyl pyrollidine), 0.2% SDS and 100 μg/ml denatured salmon sperm DNA at 41°C overnight. RNA is hybridized in the same solution at 41°C overnight with approximately 5 x 10$^6$ cpm/ml [$^{32}$P]-labelled SAP-35 cDNA. Following hybridization, filters can be washed preferably four times at room temperature with 2 x SSC, 0.2% SDS, once at 41°C with the same solution and once with 0.2 x SSC, 0.2% SDS at 50°C. Filters can then be placed between two sheets of Saran Wrap and exposed to Kodak XAR-2 film.

## Purification of SAP-35 Using an α-Methyl-Mannose-Agarose Affinity Column

Near confluent NCI-H441-4 cells are cultured in a 150cm² plastic culture flask in RPMI medium containing 25mM HEPES (pH 7.0) in 10% fetal calf serum and 20mg/l ascorbic acid. Media is removed and replaced with Dulbecco's modified Eagles media containing 1mg/ml methionine and 20mg/l ascorbic acid. 2.5 mCi of [$^{35}$S]-methionine is added to 12ml of media and the cells are incubated for about 6 hours at about 37°C, 5% $CO_2$/air. Media is collected, centrifuged at 800 rpm for about 8 minutes in a low speed centrifuge to remove cells or debris. The labelled media is dialyzed overnight at about 40°C in 4 liters of 20mM Tris-HCl, 0.1M NaCl buffer (pH 7.4). Dialysis of the labelled media is repeated two times in the same buffer the next day. The protein is added to an affinity column (11 x 1.5cm) of α-methyl-mannose-agarose (Sigma, Inc.) in the same buffer with the addition of 5mM $CaCl_2$ at room temperature. The column is washed with 30 (1ml) fractions of the buffer, then with the above buffer containing 5mM EDTA, but free of $CaCl_2$. The SAP-35 protein is eluted with the same buffer containing 0.5M mannose (10ml) which is detected in approximately the first 3mls of the mannose containing eluate, Fig. 11b. The SAP-35 protein is detected by immunoblot in the mannose fractions but is not detected in other column fractions. The [$^{35}$S]-methionine labelled proteins were then subjected to SDS-PAGE (13%), separated by electrophoresis, transferred to nitrocellulose applied to x-ray film for autoradiography, Fig. 11A. The SAP-35 proteins and its dimer, Mr = 30-35,000 and Mr = 60-65,000, respectively, were selectively eluted with the mannose containing buffer.

Isolation of Mr = 40-46,000 and Mr = 22-33,000 SPL-(Phe) Protein Precursors

Near confluent H441-4 cells are treated with 10nM dexamethasone in RPMI medium, without fetal calf serum for approximately five days. Media is removed and replaced with DMEM containing 0.5mg/l methionine in a 75cm$^2$ plastic flask incubated with 20ml of media containing 1mCi of [$^{35}$S]-methionine and incubated overnight (18 hours) at 37°C with 5% $CO_2$/air. Media (20ml) is decanted, concentrated, lyophylized to dryness and reconstituted in 1ml solubilization buffer: 190mM NaCl, 6mM EDTA, 60mM Tris-Hcl, 4% SDS and 1ml of dilution buffer: 190mM NaCl, 6mM EDTA, 50mM Tris-HCl (7.4) and 2.5% Triton X-100. The material is centrifuged in a Microfuge-B for 2 minutes, the supernatant decanted and 50 μl of rabbit anti-surfactant proteolipid (rabbit #10) is added to the supernatant to bind the SPL(Phe) precursors. After incubation overnight (18 hours) at 4°C, the sample is centrifuged 2 minutes in a Microfuge-B and the soluble protein is added to 200 μl of a 1:1 protein A-sepharose solution (Sigma Chem. Co.). The sample is rotated at room temperature for about 3 hours and washed in buffer 1 (150mM NaCl, 50mM Tris-Hcl (7.5), 5mM EDTA, 0.1% Triton and 0.02% SDS), 1mM PMSF. Protein A-sepharose complex is washed three times with 1 ml and twice with 150mM NaCl, 50mM Tris-HCl (7.5) and 50mM EDTA (buffer 2), 1mM PMSF. The washed immunoprecipitate is separated by SDS-PAGE (13%) and a portion of the gel transblotted to nitrocellulose and exposed to x-ray film for 18 hours to identify the Mr = 27-33,000 and 40-46,000 SPL(Phe) proteins, Fig. 10. The proteins are excised from the gel and subjected to electroelution in Tris borate (20mM), 0.5mM EDTA (pH 8.2) buffer with a Schleicher and Schuell electro-separation system known under the trademark as Elutrap. The purified proteins are then lyophilized and resuspended in Laemmlei cocktail and separated on 13% polyacrylamide gels, transblotted to nitrocellulose, dried and subjected to autoradiography for 72 hours at -70°C. Molecular weights are estimated by relative migration compared to standard prestained protein markers (BRL Incorporated). The purified proteins are marked Mr = 27,000 and Mr = 43,000 corresponding to the relative migration of the SPL(Phe) precursors of Mr = 27-33,000 and 40-46,000, respectively.

Regulation of Cells of NCI-H441-4 Cell Line with 8-Br-cAMP

H441-4 cells are near confluent in 35mM plastic wells and are cultured for 3 days in 100 μM 8-Br-cAMP, RPMI-10% fetal calf serum. Media is replaced with DMEM media with 1mg/ml bovine serum albumin and preincubated for about 1 hour at 37°C, 5% $CO_2$/air with or without 100 μm 8-Br-cAMP (Sigma) and 1mg/l methionine. The cells are then labelled in separate wells and processed as individual experiments. Cells are then labelled by addition of 100 μCi [$^{35}$S]-methionine for 6 hours at 37°C, 5% $CO_2$/air. Media is harvested and total methionine labelled protein assessed by TCA precipitation, normalized for immunoprecipitation of SAP-35 using rabbit-human SAP-35 serum (rabbit 361). The immunoconjugate is collected using protein A-sepharose CL-4B immunoprecipitation buffer. The proteins are transferred to nitrocellulose and subjected to autoradiography. In Fig. 5, lanes 1-3 are control (untreated) wells, lanes 4-6 are treated with 100 μM 8-Br-cAMP. Standard marks are in far right lane.

NCI-H441-4 Cell Culture

NCI-H441-4 lung adenocarcinoma cells have been isolated from a papillary adenocarcinoma of a human lung with pericardial invasion at the National Cancer Institute, Bethesda, Maryland. Tissue source was pericardial fluid. The patient was a 33 year old white male.

The original cell line culture designated as NCI-H441 was initiated on June 25, 1982 and was cultivated in ACL-3 medium, Brower et al: Growth of Cell Lines and Clinical Specimens of Human Non-Small Cell Lung Cancer in a Serum-Free Defined Medium, Cancer Res., 46:798-806 (Feb., 1986). A subline of the NCI-H441 cell line was started and maintained in the RPMI-1640 medium containing 10% fetal bovine serum. This subline is designated as NCI-H441-4, which is the same cell line as the cell line NCI-H441-4 referred to herein. As indicated, the NCI-H441-4 cell line grows as large epithelial-like cells adherent to the substrate, and as shown in Fig. 1A microscopy reveals lipid inclusions and occasional cytoplasmic multi-lamellar bodies by electron microscopy (not shown). The cells of this cell line tested positive for SAP-35 and SPL(Phe) by an ELISA type assay.

The cells of the NCI-H441-4 cell line are incubated at 37°C and 5% $CO_2$/air in RPMI-1640 in 10% fetal bovine serum with 100U/ml penicillin, 0.25 μg/ml amphotericin and 100 μg/ml streptomycin (GIBCO, Grand Island, NY). The cells are maintained in tissue culture flasks and media is changed every 6-7 days. The cells are routinely passaged every one or two weeks. Dexamethasone (Sigma Chemical Company, St. Louis, MO) can be prepared as a 10 mM stock in 95% ethanol and diluted to the stated concentration in media. For [35S]-methionine labelling, the cells are incubated in methionine-deficient (1 mg/liter) Dulbecco's Modified Eagles media with or without 0.1% bovine serum albumin replacing fetal bovine serum for metabolic labelling studies. After a one hour preincubation period, [35S]-methionine (New England Nuclear, Boston, MA), specific activity approximately equal to 1000 mCi/mole, can be added to a final concentration of 50-100 μCi/ml, and the cells incubated thereafter for 6-8 hours. Media can then be collected and proteins precipitated by the addition of iced acetone. Cells can be harvested by scraping from the plastic surfaces in a liquid mixture of 150 mM NaCl, 10 mM EDTA and 30 mM Tris, pH = 7.4 and centrifuging them at 800 g for 5 minutes. The cells can then be solubilized in 190 mM NaCl, 6 mM EDTA, 60 mM Tris-HCl pH 7.4 and 4% SDS followed by sonication for 10 seconds. After heating the lysate for 4 minutes at 100°C, an equal volume of water is added and the samples can be stored at 4°C until analysis. Two aliquots of cells and media can be routinely precipitated with 8% trichloroacetic acid for quantitation of [35S]-methionine labelling. Incorporation of [35S]-methionine into total cell protein should be normalized among samples prior to immunoprecipitation to compare treatment requirements.

Examples of purification of proteins that are synthesized by the NCI-H441-4 cell line from medium using immunoaffinity purification of the [35S]-methionine-labelled cultures and data regarding quantification are included in the Figs. 4A, 4B, 11A and 11B. Comparative induction with dexamethasone and 8-Br-cAMP are also included in Figs. 2A, 2B, 5, 6A, 6B, 7, 8, 12 and 13.

### NCI-H820 Cell Line

NCI-H820 lung adenocarcinoma cells have been initially isolated from a human bronchioloalveolar lung carcinoma metastatic to a supraclavicular lymph node at the National Cancer Institute, Bethesda, Maryland. The patient was a 50 year old white male.

The cell line culture designated as NCI-H820 was initiated on September 26, 1984, it was disaggregated mechanically, and it has been cultured continuously in ACL-4 medium, Gazdar A.F. and Oie, H. B.: Letter to Editor, Cancer Research, 46:6011-6012 (Nov., 1986). The NCI-H820 cell line lacks substrate adhesion and grows as floating cell masses with evidence of glandular formation. Like with the NCI-H441-4 cell line, microscopy reveals many cytoplasmic lipoidal inclusions as shown in Fig. 1B. In addition, the NCI-H820 cell line expresses some of the markers characteristic of neuroendocrine cells including the enzymes L-dopa decarboxylase and neuron specific enolase. The cells of this cell line tested positive for SAP-35 by an ELISA type assay. [35S]-Methionine incorporation confirmed production of SAP-35 and SPL(Phe) by the NCI-H820 cell line although at lower levels than that observed in the NCI-H441-4 cell line. Comparative induction of SPL(Phe) mRNA is shown in Fig. 9.

### NCI-H1404 Cell Line

NCI-H1404 lung adenocarcinoma cells have been initially isolated from a bronchioloalveolar carcinoma metastatic to a supraclavicular lymph node of a human lung at the National Cancer Institute, Bethesda, Maryland. The patient was a 48 year old white male.

The cell line culture designated at NCI-H1404 was initiated on June 13, 1986, it grows as non-adherent floating gland forming cell aggregates, and it is cultured continuously in ACL-4 medium. As illustrated in Fig. 1C, microscopy does not reveal granules or bodies characteristic of normal Clara cells or Type II pneumocytes. The cells of this cell line tested positive for SAP-35 by an ELISA type assay. SPL(Phe) synthesis was not detected and it is believed that the SAP-35 ELISA type assay result may represent an immunologically related protein rather than normal SAP-35.

The NCI-H441-4 cell line of the present invention will be further illustrated with reference to the following example.

## NCI-H322 Cell Line

NCI-H322 cell line was initiated on February 25, 1981 at the National Cancer Institute, Bethesda, Maryland from a human supraclavicular lymph node containing metastatic lung carcinoma. The patient was previously diagnosed and treated for small cell carcinoma of the lung in 1979. The supraclavicular lymph node components contained both small and "non-small" cell carcinoma components. In culture, only the "non-small" cell carcinoma components grew. The patient was a male.

The tumor was disaggregated mechanically, and it has been cultured continuously in RPMI 1640 medium with 10% fetal bovine serum. The NCI-H322 cell line forms adherent epithelial like cell cultures. Microscopy reveals many cytoplasmic lipoidal inclusions as shown in Fig. 1D. The cells of this cell line tested positive for SAP-35 by an ELISA type assay. [$^{35}$S]-Methionine incorporation confirmed production of a SPL(Phe) protein by the NCI-H322 cell line.

The NCL-H322 cell line tested positive for SAP-35 expression by an ELISA type assay and [$^{35}$S]-methionine incorporation demonstrated synthesis of SPL(Phe) precursor Mr = 40-46,000 by this cell line.

## EXAMPLE

## NCI-H441-4 Cell Line

The cells of the NCI-H441-4 cell line, Fig. 1A, described herein tested positive for SAP-35 and SPL(Phe) by ELISA type assays and were further studied to optimize conditions for surfactant protein production and to characterize its synthesis and structure. The cells were labelled with [$^{35}$S]-methionine and tested by immunoprecipitation assay for detection of surfactant-associated protein synthesis using anti-SAP-35 or anti-surfactant proteolipid which recognizes both SPL(Phe) and SPL-

(pVal), Figs. 3A, 3B, 5, 6A, 6B, 7 and 10. The cells were grown in monolayer cultures in 5% $CO_2$/air in RPMI media containing 10% fetal calf serum. The cells were utilized directly by harvesting and homogenization in buffer using a sonicator probe in the presence of the detergent NP-40. Conditioned media from the cultures were collected for assay of SAP-35. 8-Bromo-derivative of cAMP (100 $\mu$M) and dexamethasone (10 nM) were utilized to selectively alter the synthesis of the proteins, Figs. 2A, 2B, 5, 6A, 6B, 7 and 12. 8-Br-cAMP enhanced SAP-35 and RNA synthesis and secretion but did not enhance SPL(Phe) or SPL (Phe) RNA synthesis significantly, Fig. 5. Surprisingly, SPL(Phe) precursor and mRNA synthesis was less evident (as with other cell lines described herein) unless the tumor was pretreated with dexamethasone, Figs. 2A, 2B, 6A, 6B, 12 and 13 . The cells were therefore treated with dexamethasone for 48-72 hours prior to removal of the media and adding serum-free media to the cultures containing [$^{35}$S]-methionine for labelling of the proteins, Figs. 2A, 2B, 6A and 6B. Dexamethasone (10 nM) decreased SAP-35 and SAP-35 mRNA synthesis and secretion but markedly increased SPL(Phe) precursors and SPL-(Phe) mRNA production, Figs. 2A, 2B, 6A, 6B, 12 and 13. The proteins in the medium displayed migration in 1D and 2D-SDS-PAGE characteristic of the SPL(Phe) precursors identified in lung explants of normal human lung, Figs. 2A, 2B, 6A and 6B. The identification of size and charge, and glycosylation patterns demonstrated that the proteins were processed by the addition of N-linked carbohydrate as known for these proteins as isolated from the normal lung and from those isolated from human surfactant or as predicted from the consensus sequence predicting N-linked glycosylation derived from the base sequence of cDNA's encoding SPL(Phe) and SAP-35, Figs. 2A, 2B, 3A, 3B, 5, 6A and 6B. The SPL(Phe) or SAP-35 proteins were sensitive to endoglycosidase-F, demonstrating the presence of N-linked glycosylation on both proteins, Fig. 7. 2D-gel electrophoresis of the conditioned media, either with or without immunoprecipitation, demonstrated charge heterogeneity identical to that defined for SAP-35 in the airway of human lung or that obtained from alveolar proteinosis, Figs. 3A, 3B, 4A and 4B . Virtually identical migration of the SPL(Phe) labelled precursors was observed as described for normal human lung explant tissue, Figs. 2A, 2B, 6A, 6B and 10. The synthesis of SPL(Phe) and SAP-35 mRNA was further confirmed by using cDNA probes specific for each peptide, Fig. 8 and 13. These probes selectively recognized the mRNA's encoding for each protein and confirmed the hormonal conditions to enhance production, Fig. 8 and 13. The mRNA'S for the human SAP-35 and SPL(Phe) proteins were present in the NCI-H441-4 cells and were indistinguishable in size to those previously identified in human lung, Fig. 8.

Therefore, it is intended that the invention not be limited to the particular embodiments disclosed as the best mode contemplated for carrying out this invention, but that the invention will include all embodiments falling within the scope of the appended claims.

## Claims

1. An established human cell line comprised of epithelial-like cells which produce at least one surfactant-associated protein.

2. An established human cell line of Claim 1, wherein said cell line is derived from human epithelial lung adenocarcinoma cells.

3. An established human cell line of Claim 1, wherein said cell line has the ability to selectively express at least one surfactant-associated protein in increased amounts when exposed to an effective regulating agent in a suitable amount.

4. An established human cell line of Claim 1, wherein said cell line selectively produces SAP-35 surfactant-associated protein in increased amounts when exposed to an effective amount of 8-Br-cAMP or a functional equivalent.

5. An established human cell line of Claim 1, wherein said cell line selectively produces SPL-(Phe) surfactant-associated protein in increased amounts when exposed to an effective glucocorticoid hormone or a functional equivalent in a suitable amount.

6. An established human cell line of Claim 1, wherein said cell line is selected from a group of cell lines consisting of NCI-H441-4, NCI-H820 and NCI-H322 cell lines.

7. An established human cell line of Claim 1, wherein said cell line is NCI-H1404 cell line.

8. An established human cell line as deposited with the American Type Culture Collection (ATCC) under accession number ATCC CRL 9400.

9. An established human cell line as deposited with the American Type Culture Collection (ATCC) under accession number ATCC CRL 9531.

10. An established human cell line as deposited with the American Type Culture Collection (ATCC) under accession number ATCC CRL 9532.

11. An established human cell line as deposited with the American Type Culture Collection (ATCC) under accession number ATCC CRL 9533.

12. A method of producing a surfactant-associated protein comprising:
cultivating an established human cell line having the ability to produce at least one surfactant-associated protein in a suitable medium for a sufficient length of time to produce the surfactant-associated protein.

13. A method of Claim 12 which includes the further step of harvesting the expressed surfactant-associated protein from cell lysates of the established human cell line.

14. A method of Claim 12 which includes the further step of harvesting the expressed surfactant-associated protein from the medium.

15. A method of regulating expression of a surfactant-associated protein or an mRNA encoding such protein by cells cultured in medium and having the ability to express at least one surfactant-associated protein or mRNA encoding such protein, the cultured cells having the further ability to regulate the expression, said method comprises:
exposing the cultured cells to an effective regulating agent in an effective amount to regulate the expression.

16. A method of Claim 15, including the further step of adding the regulating agent to the medium.

17. A method of Claim 15, including the further step of transfecting or infecting the cultured cells with the regulating agent when the regulating agent is an effective gene or virus, respectively.

18. A method of Claim 15, wherein the regulating agent inhibits expression of at least one surfactant-associated protein by the cultured cells.

19. A method of Claim 15, wherein the regulating agent induces expression of at least one surfactant-associated protein by the cultured cells.

20. A method of Claim 19, wherein the regulating agent is 8-Br-cAMP or a functional equivalent for inducing expression of SAP-35 surfactant-associated protein.

21. A method of Claim 19, wherein the regulating agent is an effective glucocorticoid hormone or a functional equivalent for inducing expression of SPL(Phe) surfactant-associated protein.

22. A method of Claim 15, wherein the cultured cells are cells of an established human cell line designated as NCI-H441-4.

23. A method of Claim 15, wherein the cultured cells are cells of an established human cell line selected from a group of cell lines consisting of NCI-H820 and NCI-H322.

24. A method of harvesting a surfactant-associated protein expressed by cells cultured in medium comprising:
applying the medium or lysates of the cells to a column to which is conjugated an affinity agent for binding the surfactant-associated protein thereto, and
separating the bound surfactant-associated protein from the column to generate the surfactant-associated protein in substantially purified form.

25. A method of Claim 24, wherein the affinity agent is an anti-surfactant-associated protein antibody selected from a group consisting of polyclonal and monoclonal antibody.

26. A method of Claim 25, wherein the polyclonal or monoclonal antibody is selected from a group consisting of an anti-SPL(Phe) and anti-SPL-(pVal) antibody.

27. A method of Claim 25, wherein the polyclonal or monoclonal antibody is an anti-SAP-35 antibody.

28. A method of Claim 25, wherein the polyclonal or monoclonal antibody is an anti-surfactant-associated proteolipid antibody.

29. A method of Claim 24, wherein said separating step comprises:
eluting the column with an effective agent to remove the bound surfactant-associated protein from the column.

30. A method to screen for tumor cells of pulmonary epithelial origin that produce a surfactant-associated protein comprising:
exposing lysates of the cells or medium in which the cells are cultured to an anti-surfactant-associated protein antibody to immunoprecipitate the surfactant-associated protein produced by the tumor cells, and
identifying the immunoprecipitated protein to confirm production of the surfactant-associated protein by the tumor cells.

31. A method of Claim 30, wherein the anti-surfactant-associated protein antibody is selected from a group consisting of polyclonal and monoclonal antibody.

32. A method of Claim 30, wherein the antibody is selected from a group consisting of an anti-SPL(Phe) and SPL(pVal) antibody.

33. A method of Claim 30, wherein the antibody is an anti-SAP-35 antibody.

34. A method of Claim 30, wherein the antibody is an anti-surfactant-associated proteolipid antibody.

35. A method of Claim 30, including the further step of radio-labelling the surfactant-associated protein with $[^{35}S]$-methionine before said exposure step.

36. A method of Claim 35, wherein said identifying step comprises:
electrophoretically migrating the immunoprecipitated radio-labelled surfactant-associated protein on an electrophoretic gel, and
generating an autoradiogram of the immunoprecipitated radio-labelled surfactant-associated protein on the gel to identify the surfactant-associated protein and confirm production of the surfactant-associated protein by the tumor cells.

37. A method to screen for tumor cells of pulmonary epithelial origin that express an mRNA which codes for a surfactant-associated protein comprising:
hybridizing the mRNA isolated from the tumor cells with a radio-labelled cDNA probe complimentary to the mRNA, and
identifying the mRNA hybridized with the radio-labelled cDNA probe to confirm expression of the mRNA by the tumor cells.

38. A method of Claim 37, wherein the cDNA probe is selected from a group consisting of cDNA probes which are complimentary to mRNAs which code for a SAP-35, SPL(Phe) and SPL(pVal) precursor protein.

39. An isolated human surfactant-associated protein, said isolated human protein being a precursor of SPL(Phe) human protein and having an Mr = about 40-46,000 on SDS-PAGE and at least one of the following characteristics:
  a) sensitive to endoglycosidase-F digestion;
  b) sensitive to neuraminidase digestion;
  c) glycosylated at asparagine residues;
  d) pI is about 4.6-5.4;
  e) reactive with anti-surfactant-associated proteolipid antibody; and
  f) sialylated.

40. An isolated human surfactant-associated protein, said isolated protein being a precursor of SPL(Phe) human protein and having an Mr = about 27-33,000 on SDS-PAGE and the following characteristics:
  a) sensitive to endoglycosidase-F digestion;
  b) sensitive to neuraminidase digestion;
  c) glycosylated at asparagine residues;
  d) pI is about 6.0-7.3;
  e) reactive with anti-surfactant-associated proteolipid antibody; and
  f) sialylated.

41. An isolated human surfactant-associated protein, said isolated protein being a precursor of SPL(Phe) human protein and having an Mr = about 23,000 on SDS-PAGE and the following characteristics:
  a) de-glycosylated at asparagine residues;
  b) de-sialylated;
  c) pI is about 6.3-7.3;
  d) reactive with anti-SPL(Phe) protein antibody; and
  e) reactive with anti-surfactant-associated proteolipid antibody.

FIG. IA

FIG. IB

FIG. IC

EP 0 313 224 A1

FIG.ID

Control

FIG. 2A

Dexamethasone

FIG. 2B

FIG.3A

FIG.3B

EP 0 313 224 A1

FIG.4A

FIG.4B

FIG. 5

46 -

Mr
x 10$^{-3}$

30 -

- SP-A

1    2        3    4

# FIG. 6A

46 -

Mr
x 10$^3$

30 -

- proSP-B

5    6        7    8

# FIG. 6B

|  | 1 | 2 | 3 | 4 | 5 | 6 |
|---|---|---|---|---|---|---|
| endo-H | − | + | − | − | − | − |
| endo-F | − | − | + | − | − | + |
| collagenase | − | − | − | + | − | − |

-46
-40
-30
-23

Mr x 10³

FIG.7

kb

FIG.8

1 2   3 4

SPL (Phe)
Pheprobe

NCI-H820     NCI-H1404

<2.0

1 2     3 4

FIG.9

-3.8

-2.2
-2.0

# FIG. 10

SPL(PHE)

**PURIFICATION OF SAP 35**

start 1 5 10 15 20 30 EDTA mannose 50

fraction

## FIG. IIA

**Man-Agarose Elution**

EDTA MANNOSE

Fraction #

## FIG. IIB

Percent Maximum

Time (hr)

**FIG.13**

Percent Maximum

Log [Dexamethasone (nM)]

**FIG.12**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,P | CHEMICAL ABSTRACTS, vol. 109, no. 9, 29th August 1988, page 88, no. 67126h, Columbus, Ohio, US; M.A. O'REILLY et al.: "Differential effects of glucocorticoid on expression of surfactant proteins in a human lung adenocarcinoma cell line", BIOCHIM. BIOPHYS. ACTA 1988, 970(2), 194-204 * Abstract * | 1-39 | C 12 N 5/00 C 07 K 15/00 G 01 N 33/574 |
| X | THE JOURNAL OF BIOLOGICAL CHEMISTRY, vol. 262, no. 20, 15th July 1987, pages 9808-9811, The American Society of Biological Chemists, Inc., US; K.A. JACOBS et al.: "Isolation of a cDNA clone encoding a high molecular weight precursor to a 6-kDa pulmonary surfactant-associated protein" * Front page, abstract; page 9809, figure 1; page 9809, column 2, lines 7-17 * | 39 | |
| X,D | PROCEEDINGS OF THE NATIONAL ACADEMY OF SCIENCES, USA, vol. 84, June 1987, pages 4007-4011; S.W. GLASSER et al.: "cDNA and deduced amino acid sequence of human pulmonary surfactant-associated proteolipid SPL(Phe)" * Front page, abstract * -/- | 39 | |

**TECHNICAL FIELDS SEARCHED (Int. Cl.4)**

C 12 N
C 12 P

The present search report has been drawn up for all claims

| Place of search | Date of completion of the search | Examiner |
|---|---|---|
| THE HAGUE | 24-01-1989 | FERNANDEZ Y BRANAS F.J. |

EPO FORM 1503 03.82 (P0401)

European Patent
Office

EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | THE JOURNAL OF BIOLOGICAL CHEMISTRY, vol. 262, no. 11, 15th April 1987, pages 5256-5261, The American Society of Biological Chemists, Inc., US; J.A. WHITSETT et al.: "Induction of surfactant protein in fetal lung" * Front page, abstract * | 15-23 | |
| A,D | CANCER RESEARCH, vol. 46, February 1986, pages 798-806; M. BROWER et al.: "Growth of cell lines and clinical specimens of human non-small cell lung cancer in a serum-free defined medium" * Front page, abstract * | 1-14 | |
| A | WO-A-8 702 037 (GENETICS INSTITUTE INC.) * Page 38, claim 2 * | 39-41 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 24-01-1989 | FERNANDEZ Y BRANAS F.J. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P0401)